(19) Europäisches Patentamt
European Patent Office
Office européen des brevets

(11) EP 2 910 557 A1

(12) EUROPEAN PATENT APPLICATION

(43) Date of publication:
**26.08.2015 Bulletin 2015/35**

(51) Int Cl.:
***C07F 5/02*** *(2006.01)* ***A61P 35/00*** *(2006.01)*
***A61K 31/69*** *(2006.01)*

(21) Application number: **14382058.7**

(22) Date of filing: **20.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Ikerchem, S.L.**
**20009 San Sebastian - Guipuzcoa (ES)**
- **Universidad del Pais Vasco**
**48940 Leioa Vizcaya (ES)**

(72) Inventors:
- **Cossio Mora, Fernando Pedro**
**E-48940 Leioa-Vizcaya (ES)**
- **Retamosa Hernandez, María de Gracia**
**E-48940 Leioa-Vizcaya (ES)**
- **Larumbe Garata, Amaia**
**E-48940 Leioa-Vizcaya (ES)**
- **Zubia Olascoaga, Aizpea**
**E-48940 Leioa-Vizcaya (ES)**
- **Bello Iglesias, Tamara**
**E-48940 Leioa-Vizcaya (ES)**
- **Vara Salazar, Yosu Ion**
**E-20009 San Sebastián- Gipuzkoa (ES)**
- **Masdeu Margalef, María del Carmen**
**E-20009 San Sebastián- Gipuzkoa (ES)**
- **Aldaba Arevalo, Eneko**
**E-20009 San Sebastián- Gipuzkoa (ES)**

(74) Representative: **ABG Patentes, S.L.**
**Avenida de Burgos, 16D**
**Edificio Euromor**
**28036 Madrid (ES)**

# (54) Enantiopure tetrasubstituted pyrrolidines as scaffolds for proteasome inhibitors and medicinal applications thereof

(57) The present invention refers to compounds of general formula (I):

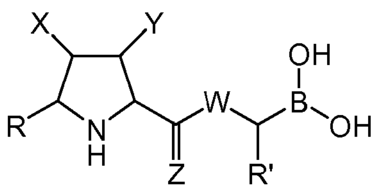

(I)

as well as to a method for their preparation, pharmaceutical compositions comprising the same, and the use thereof for the treatment and/or chemoprevention of cancer, hematological malignancy, proliferative diseases, autoimmune diseases, neurodegenerative diseases and viral infections.

EP 2 910 557 A1

## Description

### FIELD OF THE INVENTION

The present invention is related to new compounds derived from enantiopure tetrasubstituted 2-carboxy pyrrolidines for their use as proteasome inhibitors and therapeutic agents for preventing or treating pathologies such as cancer, autoimmune diseases, neurodegenerative diseases or viral infections.

### BACKGROUND OF THE INVENTION

The levels in cells of regulatory proteins are determined by a very precise balance between their synthesis and degradation. In eukaryotic cells, most proteins are degraded by the ubiquitin-proteasome (UPS) system (cf. Groll et al. ChemBioChem 2005, 6, 222-256). This pathway employs a complex supramolecular machinery known as the 26S proteasome (cf. Ciechanover Angew. Chem. Int. Ed. 2005, 44, 5944-5967; Biel et al. Angew. Chem. Int. Ed. 2004, 43, 6414-6416). This aggregate of different proteins is composed of two 19S regulatory particles (RP) and a 20S core particle (CP). This latter unit includes different active sites, which are responsible for the proteolytic activity (cf. Marques et al. Chem. Rev. 2009, 109, 1509-1536). These active sites are denoted β1, β2 and β5, and are associated with peptidyl-glutamyl-peptide hydrolysing (PGPH) or caspase-like, trypsin-like (TL) and chymotrypsin-like (CL) activities, respectively (cf. Borissenko and Groll Chem. Rev. 2007, 107, 687-717). Given the general character of these proteolytic processes, the UPS plays a pivotal role in fundamental pathways such as cell cycle regulation, DNA repair, apoptosis, immune and inflammatory responses, cystic fibrosis and, most importantly, different types of malignancies (cf. Goldberg, J. Cell Biol. 2012, 199, 583-588). Therefore, the development of proteasome inhibitors has emerged as a very active field in Medicinal Chemistry, Pharmacology and Oncology (cf. Moore et al. Curr. Opin. Chem. Biol. 2008, 12, 434-440; Cvek and Dvorak, Drug Discov. Today 2008, 13, 716-722; Bettignies and Coux, Biochimie 2010, 92, 1530-1545; Guédat and Colland, BMC Biochem. 2007, 8 (Suppl. 1), S14; D'Alessandro et al. Recent Pat. Anti-Cancer Drug Discov. 2009, 4, 73-82; Dick and Fleming, Drug Discov. Today 2010, 15, 243-249).

The different types of proteasome inhibitors can be classified according to different criteria. From the chemical standpoint, most of these inhibitors react with $Thr^1$ residues present in the β1, β2 and β5 units. Therefore these families of inhibitors incorporate a non-reactive part possessing several chiral centres, attached to an electrophilic unit. This latter moiety can be a boronic acid, a β-lactone, an aldehyde, an epoxide (with a contiguous carbonyl group) and a Michael acceptor such as a α,β-unsaturated sulphone or amide. Other proteasome inhibitors that do not correspond to this pattern include, among others, steroids, polyphenols, flavonoids, chromanes and hydroxyureas (cf. Rentsch et al., Angew. Chem. Int. Ed. 2013, 52, 5450-5488, and references therein). As far as the nonreactive parts of the inhibitors are concerned, these scaffolds incorporate diverse chiral heterocycles and/or amino acids in the form of linear or cyclic oligopeptides (cf. Kisselev et al. Chem. Biol. 2012, 19, 99-115).

As a consequence of the tremendous efforts arrived to the development of proteasome inhibitors, the U.S. Food and Drug Administration (FDA) approved in 2003 Bortezomib (Velcade), a peptide-boronic acid proteasome inhibitor, for the treatment of multiple myeloma and mantle cell lymphoma. More recently, in 2012, the FDA approved Carfilzomib (Kyprolis), a linear oligopeptide-epoxide proteasome inhibitor, for patients previously treated with Bortezomib and immunomodulatory compounds. Both Bortezomib and Carfilzomib are available as intravenous formulations. Other orally available proteasome inhibitors in clinical trials include MLN-9708 (a boronic ester) and oprozomib (ONX-0912, an oligopeptide epoxide). In addition, compound Marizomib (NPI-0052, a β-lactone) is currently undergoing phase I clinical trials for participants with advanced malignancies (cf. Molineaux, Clin. Cancer Res. 2012, 18, 15-20).

Despite the promising results obtained with these proteasome inhibitors, this therapeutic target poses important therapeutic issues such as resistance (cf. Kale and Moore, J. Med. Chem. 2012, 55, 10317-10327), selectivity (cf. Groll et al. J. Pept. Sci. 2009, 15, 58-66), toxicity and neurodegenerative effects (cf. Arastu-Kapur et al. Clin. Cancer Res. 2011, 17, 2734-2743). Therefore, novel proteasome inhibitors with better therapeutic profiles are desirable.

X-ray diffraction analysis of the structure of Bortezomib bound to yeast 20S proteasome reveals a covalent bond between the boron atom and the hydroxylic oxygen atom of Thr residues of β1, β2 and β5 units (cf. Groll et al. Structure 2006, 14, 451-456) as well as hydrogen bonding between the pyrazine moiety and Asp and Thr residues. The phenylalanine residue of Bortezomib does not interact with the surrounding protein, but occupies an empty hydrophobic space in a well-defined three dimensional arrangement (Figure 1A). We reasoned that conformational restriction around this phenyl residue should result in a better preorganised structure, thus minimizing the entropic penalty associated to the interaction between the proteasome inhibitor and the β1, β2 and β5 units of the 20S unit, as it is shown in Figure 1 B.

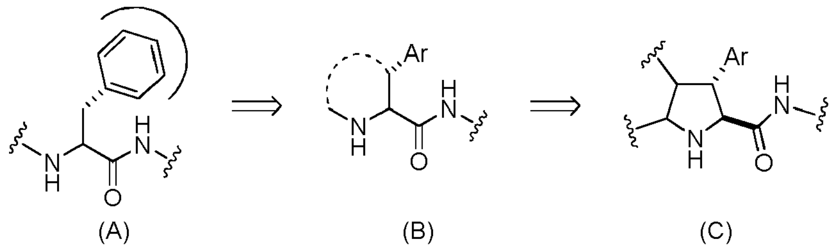

- Figure 1 -

One convenient tradeoff between conformational restriction and flexibility is provided by five membered rings. Therefore, we hypothesised that a densely substituted pyrrolidine ring scaffold possessing a carboxamide moiety at C2 and an aryl or heteroaryl group at C3 (preferably possessing R configuration, see Figure 1 C), when incorporated into a proteasome inhibitor, should improve the therapeutic profile by favorable entropic and enthalpic balance as well as by improving the selectivity of binding into different proteasome catalytic units.

Others and we have developed different methods for the regio-, diastereo- and enantiocontrolled synthesis of densely substituted pyrrolidine rings (cf. Conde et al. Chem. Sci. 2012, 3, 1486-1491; Castelló et al. Org. Lett. 2013, 15, 2902-2905). In addition, pyrrolidine rings have been described as proteasome inhibitors (cf. Adams et al. WO96/13266). However, the activity of enantiopure tetrasubstituted pyrrolidines as proteasome inhibitors has not been reported. In particular, the effect of the absolute configuration of C4 atom of the pyrrolidine ring on the inhibitory capacity of the resulting molecule is unknown.

## OBJECT OF THE INVENTION

A first aspect of the invention refers to a compound of general formula (I),

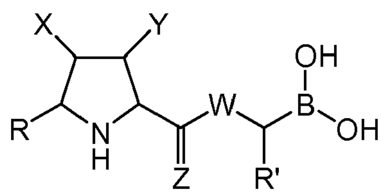

(I)

wherein:

X is an electron withdrawing group selected from $-NO_2$, $-C(O)R^1$, $-C(O)OR^1$ and $-C(O)NR^2R^3$, or forms together with Y a cycloalkyl or heterocycloalkyl group, wherein $R^1$ is an alkyl group and $R^2$ and $R^3$ are independently selected from hydrogen and alkyl;

Y is selected from aryl, heteroaryl having at least one heteroatom, and $-C(O)OR^4$ or form together with X a cycloalkyl or heterocycloalkyl group, wherein $R^4$ is an alkyl group;

R is selected from mono- or polycyclic aryl and heteroaryl having at least one heteroatom;

Z is O or S;

W is selected from -NH-, -O-, and $-CH_2-$;

R' is selected from linear or branched alkyl and cycloalkyl;

or stereoisomers, solvates, salts or prodrugs thereof.

Likewise, another aspect of the present invention refers to a process for the preparation of compounds of general formula (I), or stereoisomers, solvates, salts or prodrugs thereof, which comprises:

a) reacting an alkene of general formula (IIa) or (IIb),

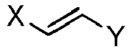

(IIa)

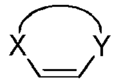

(IIb)

wherein X and Y have the meaning given above;
with an imine of general formula (III),

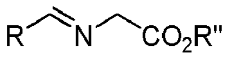

(III)

wherein R has the meaning given above and R" is a linear or branched alkyl group selected from methyl, ethyl and *tert*-butyl;
in the presence of an organic or inorganic base, an aprotic solvent and a metallic salt, to afford a compound of formula (IV):

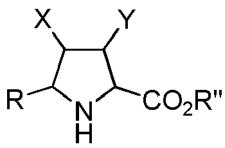

(IV)

b) reacting a compound of formula (IV) with a mixture comprising an organic solvent and:

i) an aqueous solution of an alkali or alkaline earth hydroxide; or

ii) a trialkyltin hydroxide; or

iii) an organic acid,

to afford a compound of formula (VI)

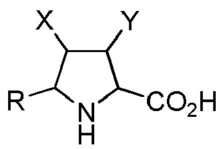

(VI)

c) reacting the compound of formula (VI) with a boronic ester of formula (VII):

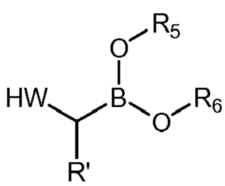

(VII)

wherein

R' and W have the meaning given above,

$R^5$ and $R^6$ are independently an alkyl group or together form an alkylene group, a cycloalkylene group or an arylene group;

the WH group can be neutral or forming a salt,

in an organic solvent in the presence of an organic base and a coupling agent,

to afford an intermediate boronic ester;

d) deprotecting the intermediate boronic ester by treatment with isobutylboronic acid and an aqueous solution of hydrochloric acid, to afford the compound of formula (I) wherein Z is O; and
e) reacting the compound of formula (I) obtained in step d) with a Lawesson reactive or a derivative thereof to afford the compound of formula (I) wherein Z is S.

Another aspect of this invention relates to a compound of general formula (I), or a stereoisomer, salt, solvate or prodrug thereof, for its use as a medicament.
Another aspect of the present invention relates to a compound of general formula (I), or a stereoisomer, salt, solvate or prodrug thereof, for its use in the treatment of cancer, hematological malignancy, proliferative diseases or inflammation disorders.
Another aspect of the present invention relates to the use of a compound of general formula (I), or a stereoisomer, salt, solvate or prodrug thereof, in the preparation of a medicament for the treatment of cancer, hematological malignancy, proliferative diseases or inflammation disorders.
According to another aspect the present invention is directed to a method of treating cancer, hematological malignancy, proliferative diseases or inflammation disorders, which comprises the administration to a patient needing such treatment, consisting of a therapeutically effective amount of at least one compound of general formula (I) or a stereoisomer, salt, solvate or prodrug thereof.
A further object of the invention described below is a pharmaceutical composition comprising at least one compound of general formula (I), or a stereoisomer, salt, solvate or prodrug thereof, and at least one pharmaceutically acceptable excipient.

**DETAILED DESCRIPTION OF THE INVENTION**

First, the present invention provides compounds of general formula (I),

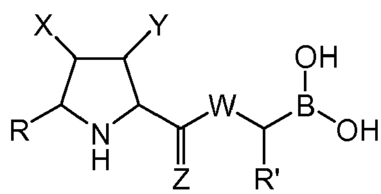

wherein:

X is an electron withdrawing group selected from $-NO_2$, $-C(O)R^1$, $-C(O)OR^1$ and $C(O)NR^2R^3$, or forms together with Y a cycloalkyl or heterocycloalkyl group, wherein $R^1$ is an alkyl group and $R^2$ and $R^3$ are independently selected from hydrogen and alkyl;

Y is selected from aryl, heteroaryl having at least one heteroatom, and $-C(O)OR^4$, or forms together with X a cycloalkyl or heterocycloalkyl group, wherein $R^4$ is an alkyl group;

R is selected from mono- or polycyclic aryl and heteroaryl having at least one heteroatom;

Z is O or S;

W is selected from -NH-, -O- and $-CH_2-$;

R' is selected from linear or branched alkyl and cycloalkyl;

or a stereoisomer, a solvate,a salt or prodrug thereof.

The term "alkyl" refers to a linear, or branched hydrocarbon chain radical consisting of carbon and hydrogen atoms, containing no unsaturation, having 1 to 8 carbon atoms, which is attached to the rest of the molecule by a single bond. Examples of such alkyl groups include, but are not limited to, methyl, ethyl, *n*-propyl, *i*-propyl, *n*-butyl, s-butyl, *i*-butyl or *tert*-butyl.

The term "cycloalkyl" means a non-aromatic, monocyclic or polycyclic ring comprising carbon and hydrogen atoms. A cycloalkyl group can have one or more carbon-carbon double bonds in the ring so long as the ring is not rendered aromatic by their presence. Examples of cycloalkyl groups include, but are not limited to, $C_3$-$C_7$ fully saturated cycloalkyl groups, such as cyclopropyl, cyclobutyl, cyclopentyl, cyclohexyl, and cycloheptyl, and saturated cyclic and bicyclic terpenes and $C_3$-$C_7$ cycloalkenyl groups, such as cyclopropenyl, cyclobutenyl, cyclopentenyl, cyclohexenyl, and cycloheptenyl, and unsaturated cyclic and bicyclic terpenes. A cycloalkyl group can be unsubstituted or substituted by one or two suitable substituents. Preferably, the cycloalkyl group is a monocyclic ring or bicyclic ring.

The term "heterocycloalkyl" means a non-aromatic monocyclic or polycyclic ring comprising carbon and hydrogen atoms and at least one heteroatom, preferably, 1 to 4 heteroatoms selected from nitrogen, oxygen, and sulfur. A heterocycloalkyl group can have one or more carbon-carbon double bonds or carbon-heteroatoms double bonds in the ring as long as the ring is not rendered aromatic by their presence. Examples of heterocycloalkyl groups include aziridinyl, pyrrolidinyl, pyrrolidino, piperidinyl, piperidino, piperazinyl, piperazino, morpholinyl, morpholino, thiomorpholinyl, thiomorpholino, tetrahydrofuranyl, tetrahydrothiofuranyl, tetrahydropyranyl, and pyranyl. A heterocycloalkyl group can be unsubstituted or substituted with one or more suitable substituents, preferably with alkyl groups and/or ketone groups. Preferably, the heterocycloalkyl group is a monocyclic or bicyclic ring, more preferably, a monocyclic ring, wherein the ring comprises from 2 to 6 carbon atoms and from 1 to 3 heteroatoms. The heterocycloalkyl group is preferably a 1-alkyl-pyrrolidine-2,5-dione, more preferably 1-methyl-pyrrolidine-2,5-dione.

The term "aryl" refers to a $C_6$-$C_{10}$ aromatic group comprising 1, 2 or 3 aromatic rings, linked by a carbon-carbon bond or condensed, optionally substituted with a group selected from a $C_1$-$C_6$ alkyl, halogen, nitro, cyano, $OR^1$, $SR^1$, $SOR^1$, $SO_2R^1$, $NR^1R^2$, $C(O)R^1$, $C(O)OR^1$, $C(O)NR^1R^2$ or $OC(O)R^1$, wherein $R^1$ and $R^2$ are hydrogen or an alkyl group as defined above. The term aryl includes, for example, and in a non-limiting sense, phenyl, napthyl, biphenyl, indenyl, etc.

The term "heteroaryl" refers to a stable monocyclic or polycyclic 3 to 10 membered aromatic ring, preferably a 5 or 6 membered aromatic ring, which consists of carbon atoms and from 1 to 5 heteroatoms selected from nitrogen, oxygen and sulfur. For the purposes of the invention, the heteroaryl radical may be optionally oxidized; and nitrogen, carbon or sulfur atoms in the heteroaryl radical may be optionally oxidized; and the nitrogen atom may be optionally quaternized. Examples of such heteroatom substituent include, but are not limited to, benzimidazole, benzothiazole, furan, thiophene, pyrrole, pyridine, pyrimidine, isothiazole, imidazole, indole, purine, quinoline and thiodiazole.

In a particular embodiment of the invention, the group W is -NH-.

In another particular embodiment, Z is O.

Even in a preferred embodiment, W is -NH- and Z s O.

In another particular embodiment, X is selected from $-NO_2$, and $-C(O)OR^1$, wherein $R^1$ is an alkyl group, or form together with Y a heterocycloalkyl group. More preferably, X is $-NO_2$, -C(O)OMe or form together with Y 1-methyl-pyrrolidine-2,5-dione.

In another particular embodiment, R' is a linear or branched alkyl group, more preferably is a branched alkyl group, even more preferably is an iso-butyl group.

In a preferred embodiment, the compounds of general formula (I) are selected from:

3-Methyl-1-[4-nitro-3,5-diphenylpyrrolidine-2-carboxamido]butyl boronic acid;

3-Methyl-1-[4-nitro-5-phenyl-3-p-tolylpyrrolidine-2-carboxamido] butylboronic acid;

1-[3-(4-Fluorophenyl)-4-nitro-5-phenylpyrrolidine-2-carboxamido] -3-methylbutylboronic acid;

1-[3-(4-Methoxyphenyl)-4-nitro-5-phenylpyrrolidine-2-carboxamido]-3-methylbutylboronic acid;

3-Methyl-1-[4-nitro-3-(4-nitrophenyl)-5-phenylpyrrolidine-2-carboxamido]butylboronic acid;

3-Methyl-1-[3-(naphthalen-2-yl)-4-nitro-5-phenylpyrrolidine-2-carboxamido]butylboronic acid;

3-Methyl-1-[4-nitro-5-phenyl-3-(thiophen-2-yl)pyrrolidine-2-carboxamido]butylboronic acid

1-[3-(Furan-2-yl)-4-nitro-5-phenylpyrrolidine-2-carboxamido]-3-methylbutylboronic acid;

3-Methyl-1-[5-(naphthalen-2-yl)-4-nitro-3-phenylpyrrolidine-2-carboxamido]butylboronic acid;

3-Methyl-1-[5-(naphthalen-1-yl)-4-nitro-3-phenylpyrrolidine-2-carboxamido]butylboronic acid;

3-Methyl-1-[5-(naphthalen-1-yl)-4-nitro-3-phenylpyrrolidine-2-carboxamido]butylboronic acid;

1-[5-(Furan-2-yl)-4-nitro-3-phenylpyrrolidine-2-carboxamido]-3-methylbutylboronic acid;

3-Methyl-1-[4-nitro-3-phenyl-5-(pyridin-3-yl)pyrrolidine-2-carboxamido]butylboronic acid;

1-[3,4-Bis(methoxycarbonyl)-5-phenylpyrrolidine-2-carboxamido]-3-methylbutylboronic acid;

3-Methyl-1-{5-methyl-4,6-dioxo-3-phenyloctahydropyrrolo [3,4-c]pyrrole-1-carboxamido}butylboronic acid

3-Methyl-1-{5-methyl-4,6-dioxo-3-phenyloctahydropyrrolo [3,4-c]pyrrole-1-carboxamido}butylboronic acid.

The compounds of the invention comprise asymmetric substituents, which may give raise to the presence of different stereoisomers (enantiomer, stereoisomers, etc). The single isomers, enantiomers or diastereoisomers, also fall within the scope of the present invention.

In a particular embodiment, the compound of formula (I) is selected from the compounds of formula (L-Ia) and formula (D-Ia):

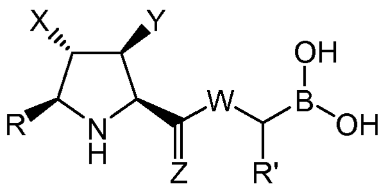

(L-Ia)

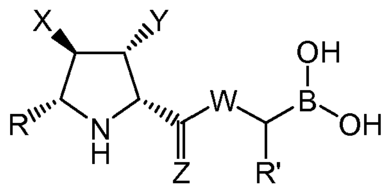

(D-Ia)

wherein:

R, R', Z and W are as defined above;

X is an electron withdrawing group selected from $-NO_2$, $C(O)R^1$, $C(O)OR^1$ and-$C(O)NR^2R^3$, wherein $R^1$, $R^2$ and $R^3$ are as defined above; and

Y is selected from aryl, heteroaryl having at least one heteroatom, and $-C(O)OR^4$, wherein $R^4$ is as defined above.

In another particular embodiment, the compound of formula (I) is selected from the compounds of formula (L-Ib) and formula (D-Ib):

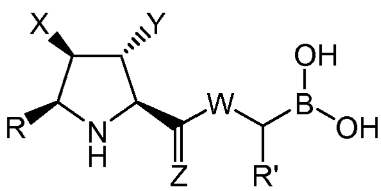

(L-Ib)

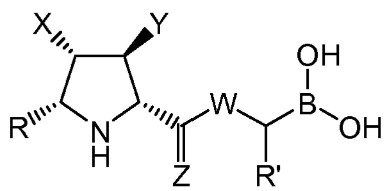

(D-Ib)

wherein:

R, R', Z and W are as defined above;

X is an electron withdrawing group selected from $-NO_2$, $C(O)R^1$, $C(O)OR^1$ and-$C(O)NR^2R^3$, wherein $R^1$, $R^2$ and $R^3$ are as defined above;

Y is selected from aryl, heteroaryl having at least one heteroatom, and -$C(O)OR^4$, wherein $R^4$ is as defined above.

In another particular embodiment, the compound of formula (I) is selected from the compounds of formula (L-Ic) and formula (D-Ic):

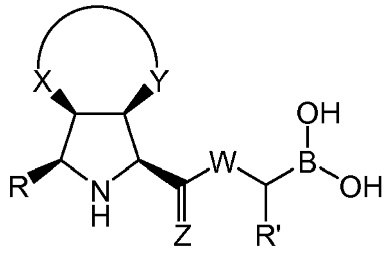

(L-Ic)

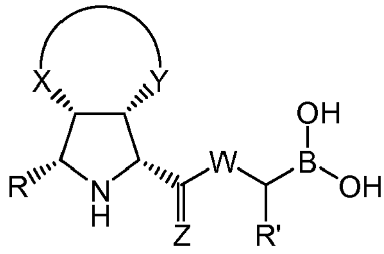

(D-Ic)

wherein

R, R', Z and W are as defined above; and

X and Y are linked to each other thus forming a cylcoalkyl or heterocycloalkyl group.

More preferable are the following stereoisomers:

[1] (*R*)-3-Methyl-1-[(2*R*,3*R*,4*S*,5*R*)-4-nitro-3,5-diphenylpyrrolidine-2-carboxamido]butyl boronic acid, with the following structural formula:

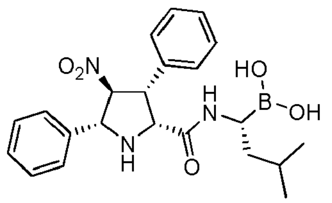

[2] (*R*)-3-Methyl-1-[(2S,3S,4R,5S)-4-nitro-3,5-diphenylpyrrolidine-2-carboxamido]butyl boronic acid, with the following structural formula:

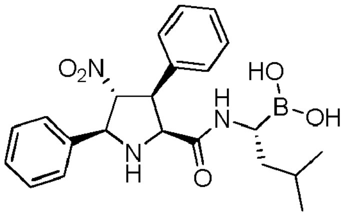

[3] (*R*)-3-Methyl-1-[(2*R*,3*S*,4*R*,5*R*)-4-nitro-3,5-diphenylpyrrolidine-2-carboxamido)butyl boronic acid, with the following structural formula:

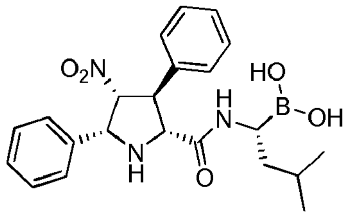

[4] (*R*)-3-Methyl-1-[(2*S*,3*R*,4*S*,5*S*)-4-nitro-3,5-diphenylpyrrolidine-2-carboxamido]butyl boronic acid, with the following structural formula:

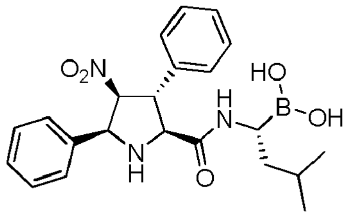

[5] (*R*)-3-Methyl-1-[(2*R*,3*R*,4*S*,5*R*)-4-nitro-5-phenyl-3-p-tolylpyrrolidine-2-carboxamido] butylboronic acid, with the following structural formula:

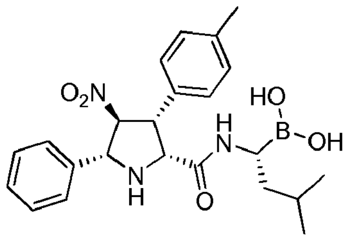

[6] (*R*)-1-[(2*R*,3*R*,4*S*,5*R*)-3-(4-Fluorophenyl)-4-nitro-5-phenylpyrrolidine-2-carboxamido] -3-methylbutylboronic acid, with the following structural formula:

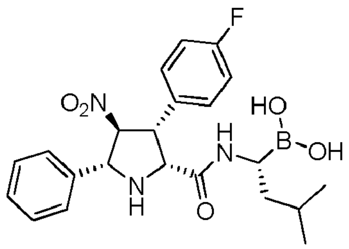

[7] (*R*)-1-[(2*R*,3*R*,4*S*,5*R*)-3-(4-Methoxyphenyl)-4-nitro-5-phenylpyrrolidine-2-carboxamido]-3-methylbutylboronic acid, with the following structural formula:

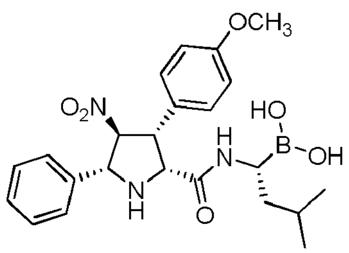

[8] (*R*)-3-Methyl-1-[(2*R*,3*R*,4*S*,5*R*)-4-nitro-3-(4-nitrophenyl)-5-phenylpyrrolidine-2-carboxamido]butylboronic acid, with the following structural formula:

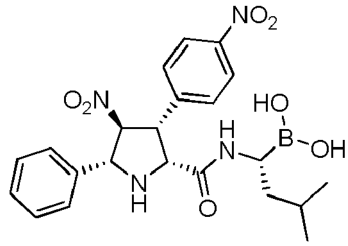

[9] (*R*)-3-Methyl-1-[(2*R*,3*R*,4*S*,5*R*)-3-(naphthalen-2-yl)-4-nitro-5-phenylpyrrolidine-2-carboxamido]butylboronic acid, with the following structural formula:

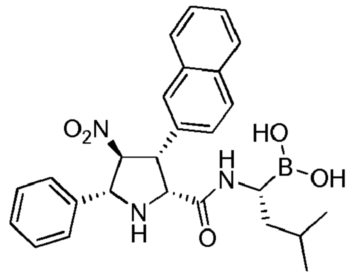

[10] (*R*)-3-Methyl-1-[(2*R*,3*R*,4*S*,5*R*)-4-nitro-5-phenyl-3-(thiophen-2-yl)pyrrolidine-2-carboxamido]butylboronic acid, with the following structural formula:

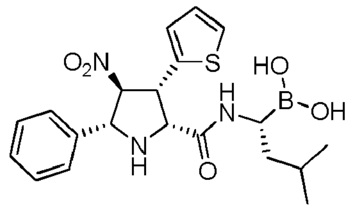

[11] (*R*)-1-[(2*R*,3*R*,4*S*,5*R*)-3-(Furan-2-yl)-4-nitro-5-phenylpyrrolidine-2-carboxamido]-3-methylbutylboronic acid, with the following structural formula:

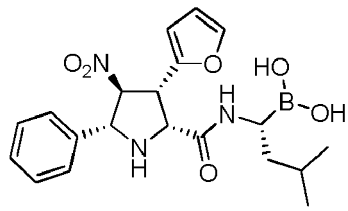

[12] (*R*)-3-Methyl-1-[(2*R*,3*R*,4*S*,5*R*)-5-(naphthalen-2-yl)-4-nitro-3-phenylpyrrolidine-2-carboxamido]butylboronic acid, with the following structural formula:

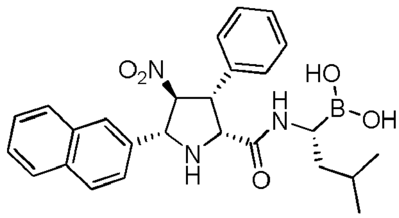

[13] (*R*)-3-Methyl-1-[(2*R*,3*R*,4*S*,5*R*)-5-(naphthalen-1-yl)-4-nitro-3-phenylpyrrolidine-2-carboxamido]butylboronic acid, with the following structural formula:

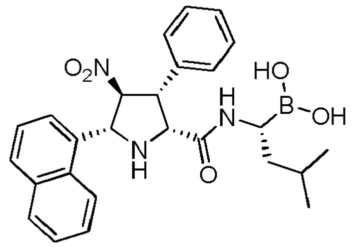

[14] (*R*)-3-Methyl-1-[(2*R*,3*S*,4*R*,5*R*)-5-(naphthalen-1-yl)-4-nitro-3-phenylpyrrolidine-2-carboxamido]butylboronic acid, with the following structural formula:

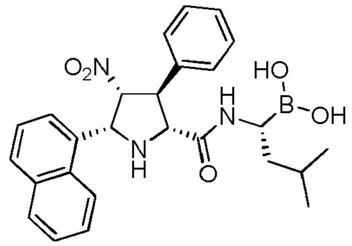

[15] (*R*)-1-[(2*R*,3*R*,4*S*,5*S*)-5-(Furan-2-yl)-4-nitro-3-phenylpyrrolidine-2-carboxamido]-3-methylbutylboronic acid, with the following structural formula:

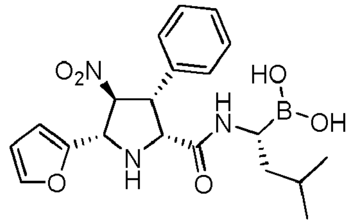

[16] (*R*)-3-Methyl-1-[(2*R*,3*R*,4*S*,5*R*)-4-nitro-3-phenyl-5-(pyridin-3-yl)pyrrolidine-2-carboxamido]butylboronic acid, with the following structural formula:

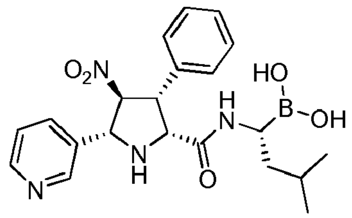

[17] (*R*)-1-[(2*S*,3*S*,4*S*,5*R*)-3,4-Bis(methoxycarbonyl)-5-phenylpyrrolidine-2-carboxamido]-3-methylbutylboronic acid, with the following structural formula:

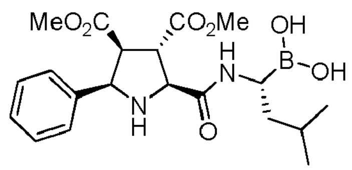

[18] (*R*)-3-Methyl-1-{(1*S*,3*R*,3a*S*,6a*R*)-5-methyl-4,6-dioxo-3-phenyloctahydropyrrolo [3,4-c]pyrrole-1-carboxamido} butylboronic acid, with the following structural formula:

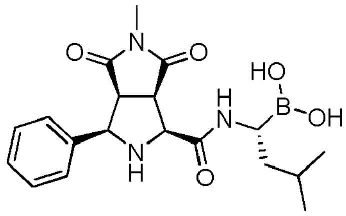

[19] (*R*)-3-Methyl-1-{(1*R*,3*S*,3a*R*,6a*S*)-5-methyl-4,6-dioxo-3-phenyloctahydropyrrolo [3,4-c]pyrrole-1-carboxamido} butylboronic acid, with the following structural formula:

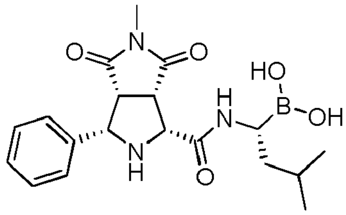

The compounds of formula (I) defined above can also be in the form of solvates or salts or prodrugs, preferably as a pharmaceutically acceptable species.

The term "pharmaceutically acceptable species" refers to solutions, suspensions, mixtures and molecular entities that are physiologically tolerable and in general do not produce an allergic reaction or similar unfavorable reaction as gastric disorders, dizziness and suchlike, when administered to a human or animal. Preferably, the term "pharmaceutically acceptable" means it is approved by a regulatory agency of a state of federal government or is included in the U.S. Pharmacopoeia or other generally recognized pharmacopoeia for use in animals, and preferably in humans.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject.. Experts in the art would readily produce such derivatives, and include, depending of the functional groups present in the molecule and without limitation, the following derivatives of the present compounds: boronic esters, esters of metallic salt sulfonates, carbonates, ureas and amides. Examples of well known methods of producing a prodrug of a given acting compound are known to those skilled in the art and can be found e.g. in Krogsgaard-Larsen et al., Textbook of Drug design and Discovery, Taylor & Francis (April 2002).

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "solvate" is to be understood as meaning any form of the active compound according to the invention which has another molecule (for example a polar solvent such as water or ethanol, a cyclodextrin or a dendrimer) attached to it through non-covalent bonds. Methods of solvation are known within the art. Examples of such solvates include hydrates and alcoholates, e.g. methanolates.

The invention also provides salts of the compounds of the invention. Non-limiting examples are sulphates; hydrohalide salts; phosphates; lower alkane sulphonates; aryl sulphonates; salts of $C_1$-$C_{20}$ aliphatic mono-, di- or tribasic acids which may contain one or more double bonds, an aryl nucleus or other functional groups such as hydroxy, amino, or keto; salts of aromatic acids in which the aromatic nuclei may or may not be substituted with groups such as hydroxyl, lower alkoxyl, amino, mono- or di- lower alkylamino sulphonamido.

Solvates, salts and prodrugs can be prepared by methods known in the state of the art. Note that the non-pharmaceutically acceptable solvates and prodrugs also fall within the scope of the invention because they can be useful in preparing pharmaceutically acceptable salts, solvates or prodrugs.

The compounds of the invention also seek to include compounds that differ only in the presence of one or more isotopically enriched atoms. For example, compounds having the present structures except for the replacement of a hydrogen by a deuterium or tritium, or the replacement of a carbon by a carbon enriched in $^{11}C$, $^{13}C$ or $^{14}C$, or $^{18}F$ and/or $^{15}N$ enriched compounds are within the scope of this invention.

<u>Synthesis of compounds of formula (I)</u>

Another aspect of the invention refers to a process to obtain compounds of general formula (I) as defined above.

Step a) of the process of the invention involves the reaction of the alkene of formula (IIa) or (IIb) with the imine of formula (III) defined above in the presence of an organic or inorganic base, an aprotic solvent and a metallic salt to afford the compound of formula (IV).

In a particular embodiment, when an enantiopure compound of formula (I) wants to be obtained, step a) is carried out also in the presence of a chiral catalyst.

As it would be evident for a skilled person, by a chiral catalyst it is understood a catalyst that allows obtaining the required stereochemistry for a particular compound.

In a particular embodiment the chiral catalyst is selected from:

| | | | |
|---|---|---|---|
| 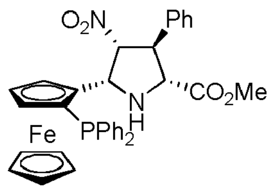<br>(D-Va) | 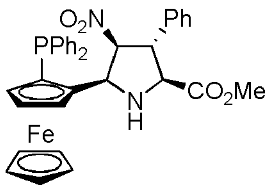<br>(L-Va) | 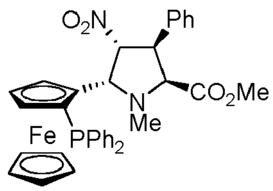<br>(L-Vb) | 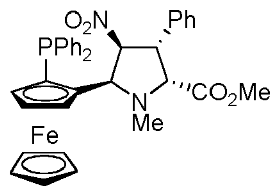<br>(D-Vb) |

The reaction mixture in step a) is generated by mixing the selected chiral catalyst, if any, with a metallic salt. Said metallic salt can be formed by a copper (I), copper (II), lithium (I), magnesium (II) or silver (I) cation, and the appropriate anion and, when possible, neutral ligands. The resulting mixture can be stirred at a temperature comprised between -70°C and +25°C. To this mixture, the alkene (IIa) or (IIb) and the imine (III), together with the organic base, can be added in any order.

The organic base may be a primary, secondary or tertiary amine, preferably a tertiary amine selected from cyclic and acyclic aliphatic amines with $C_3$-$C_{10}$.

The resulting compound can be isolated by filtration of the reaction mixture through a pad of celite, silica gel or alumina, followed by concentration under reduced pressure.

Separation and dehydration of the organic phase, followed by evaporation of the organic solvent, yields compounds of formula (IV) which can be purified by chromatography or trituration.

Step b) of the process of the invention involves the formation of carboxylic acid derivatives of formula (VI). This step can be accomplished by means of the following procedures:

i) hydrolysis of compounds of formula (IV) in the presence of an appropriate organic solvent and an aqueous solution of a hydroxide of alkali or alkaline earth metal;

ii) treatment of compounds of formula (IV) with a trialkyltin hydroxide in the presence of an appropriate organic solvent;

iii) treatment of compounds of formula (IV) with an organic acid in the presence of an organic solvent. In a preferred embodiment of the invention, the organic acid is trifluoroacetic acid and the starting ester of formula (IV) is a *tert*-butyl ester.

Step c) of the process of the invention can be carried out by adding, under argon atmosphere, the carboxylic acid of formula (VI), a coupling reagent, and the boronic ester (VII) or its hydrochloride salt to a dry organic solvent, at a temperature comprised between -100°C and +25°C. To the resulting solution or suspension a mixture of an organic base solved in an organic solvent is added at the same temperature. After completion of the reaction, a less polar organic solvent such as ethyl acetate is added and the resulting solution is washed. The organic layer is separated, dried, and concentrated under reduced pressure.

The resulting product is solved in an alcohol and an apolar solvent. The resulting solution is treated with isobutyl boronic acid and an aqueous solution of hydrochloric acid. After an appropriate reaction time at a temperature comprised between 0°C and 25°C, an appropriate solvent such as ethyl acetate is added. The organic layer is washed with a weak basic aqueous solution. The organic layer is separated and dried. Filtration and evaporation of the organic solvent yields the product (I) wherein Z is O, which can be purified by means of an appropriate method such as trituration, crystallization or chromatographic technique.

A preferred embodiment of step c) of the process of the invention includes *N,N*-dimethylformamide or dimethylsulfoxide as polar solvents.

Likewise, preferred organic bases are cyclic or acyclic aliphatic amines with $C_3$-$C_{10}$.

The coupling reagent can be chosen among the compounds reported in the literature. Preferred coupling reagents are *O*-(benzotriazol-1-yl)-*N,N,N',N'*-tetramethyluronium tetrafluoroborate, 1-[bis(dimethylamino)methylene]-1*H*-1,2,3-triazolo[4,5b]pyridinium-3-oxid hexafluorophosphate, benzotriazol-1-yl-oxytripyrrolidinophosphonium hexafluorophosphate, and pairs formed by *N,N'*-dicyclohexylcarbodiimide or *N,N'*-diisopropylcarbodiimide and 1-hydroxy-7-azabenzotriazole or hydroxybenzotriazole.

The alcohol can be selected among any alcohol liquid at room temperature.

In a particular embodiment, the boronioc ester of formula (VII) is selected from:

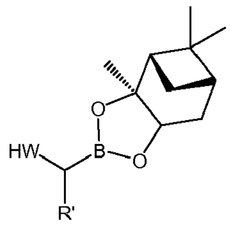
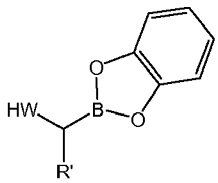
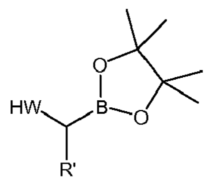

wherein R' and W are as defined above.

When it is desired to obtain a compound of formula (I) in which Z is S, the compound of formula (I) obtained above, in which Z is O, is subjected to a reaction with a Lawesson reactive or a derivative thereof following the procedures described for example by Kaleta, Z et al. (Org. Lett., 2006, 8, 1625-1628), Jones, B. A. et al. (Chem. Rev. (Review) 84 (84): 17 and Shabana, R. et al. (Nouveau Journal de Chimie 1980, (4): 47).

A further embodiment of the invention is a salt or solvate or prodrug thereof of a compound of formula (I). According to a particular embodiment, the salt is a boronate salt of alkali or alkaline earth metals. To obtain these salts, the boronic group can be treated with hydroxides of alkali or alkaline earth metals (e.g. sodium, lithium, potassium, calcium, or magnesium hydroxide) at a temperature ranging from 0°C to +40°C. In an embodiment of the invention the reaction takes place at room temperature using water as solvent.

To obtain ammonium salts corresponding to compounds of formula (I), the secondary amino group can be treated with organic or inorganic acids. In an embodiment of the invention, these acids can be selected among the usual acids of acceptable pharmacological use. To obtain these ammonium salts the amino group can be treated directly with the corresponding acid in an appropriate solvent at a temperature ranging from 0°C to +30°C. In the products thus formed, the anion associated with the corresponding salt can be chloride, acetate, tartrate, lactate, succinate, or similar.

The following methods A, B and C describe suitable procedures to obtain stereoisomer compounds of formula (L/D-Ia), (L/D-Ib), and (L/D-Ic), respectively, or solvates or salts or prodrugs thereof as defined above.

<u>Method A</u>

Method A describes the procedure for obtaining compounds of general formula (L/D-Ia),

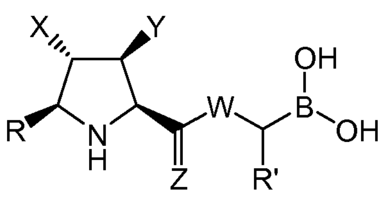

(L-Ia)

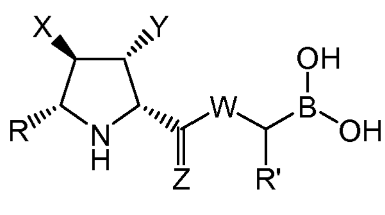

(D-Ia)

wherein X, Y, R, R', Z and W have the meaning given above and X and Y are not linked to each other, which comprises:

a) reacting an (E)-alkene of general formula (IIa),

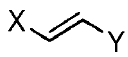

(IIa)

wherein X and Y have the meaning given above;

with an imine of general formula (III),

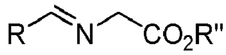

(III)

wherein R has the meaning given above and R" is a linear or branched alkyl group, preferably methyl, ethyl, or *tert*-butyl;

in the presence of an organic or inorganic base, an aprotic solvent, a metallic salt, and an appropriate chiral catalyst, to afford compounds of formula (L/D-IVa):

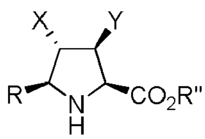

(L-IVa)

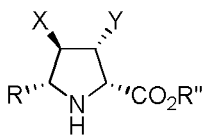

(D-IVa)

b) reacting the compounds of formula (L-IVa) and (D-IVa) in a mixture selected from:

- a polar organic solvent and an aqueous solution of a hydroxide of alkali or alkaline earth metal;
- an organic solvent and a trialkyltin hydroxide; and
- an organic acid and an organic solvent,
  to afford compounds of formula (L-VIa) and (D-VIa):

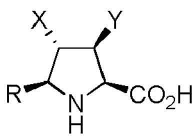

(L-VIa)

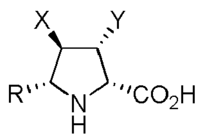

(D-VIa)

wherein R, X and Y have the meaning given above;

c) reacting the compounds of formulas (L/D-VIa) with a boronic ester of general formula (VII) or its hydrochloride salt:

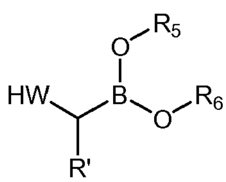

(VII)

wherein

R' and W have the meaning given above,

and

$R^5$ and $R^6$ are independently an alkyl group or together form an alkylene group, a cycloalkylene group or an arylene group;

the group WH can be neutral or forming a salt,

in the presence of an organic base, a suitable coupling reagent, and an appropriate organic solvent, to afford an intermediate boronic ester;

d) deprotecting the intermediate boronic ester by treatment with isobutylboronic acid and an aqueous solution of hydrochloric acid to afford compounds of formula (L/D-Ia) wherein Z is O, and
e) reacting the compound of formula (I) obtained in step d) with a Lawesson reactive or a derivative thereof to afford the compound of formula (L/D-Ia) wherein Z is S.

In a particular embodiment, the chiral catalyst used in step a) of the Method A to yield compounds of formula (L-IVa) is a compound of formula (D-Va):

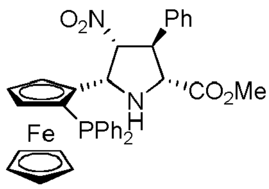

(D-Va)

In another aprticualr embodiment, the chiral catalyst used in step a) of the Method A to yield compounds of formula (D-IVa) is a compound of formula (L-Va):

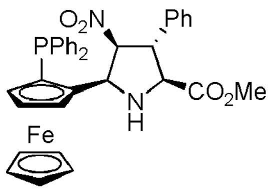

(L-Va)

Method B

Method B describes the procedure for obtaining compounds of general formula (L/D-Ib),

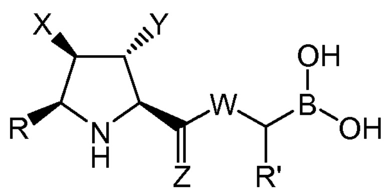

(L-Ib)

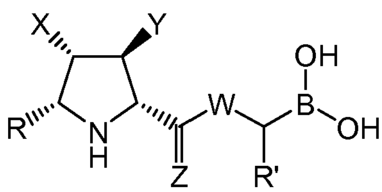

(D-Ib)

wherein X, Y, R, R', Z and W have the meaning given above and X and Y are not linked to each other, which comprises:

a) reacting an (*E*)-alkene of general formula (IIa) as described above;
with an imine of general formula (III) as described above;
in the presence of an organic or inorganic base, an aprotic solvent, a metallic salt, and an appropriate chiral catalyst, to afford compounds of formulas (L/D-IVb):

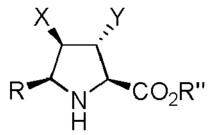
(L-IVb)

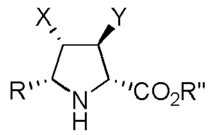
(D-IVb)

b) reacting the compounds of formula (L-IVb) and (D-IVb) in a mixture selected from:

- a polar organic solvent with an aqueous solution of a hydroxide of an alkali or an alkaline earth metal;
- an organic solvent and a trialkyltin hydroxide; and
- an organic acid and an organic solvent,
to afford compounds of formula (L-VIb) and (D-VIb):

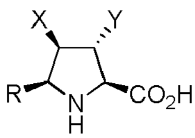
(L-VIb)

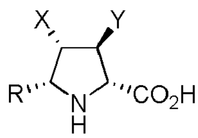
(D-VIb)

wherein R, X and Y have the meaning given above;

c) reacting the compounds of formula (L/D-VIb) with aboronic ester of general formula (VII) as described above, or its hydrochloride salt;
in the presence of an organic base, a suitable coupling reagent, and an appropriate organic solvent, to afford an intermediate boronic ester;

d) deprotecting said intermediate boronic ester by treatment with isobutylboronic acid and an aqueous solution of hydrochloric acid to afford compounds of formula (L/D-Ib) wherein Z is O; and

e) reacting the compound of formula (I) obtained in step d) with a Lawesson reactive or a derivative thereof to afford the compound of formula (L/D-Ib) wherein Z is S.

In a particular embodiment, the chiral additive of the catalytic system required to yield compounds of formula (L-IVb) is a compound of formula (L-Vb):

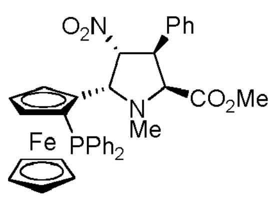
(L-Vb)

In another particular embodiment, the chiral additive of the catalytic system required to yield compounds of formula (D-IVb) is a compound of formula (D-Vb):

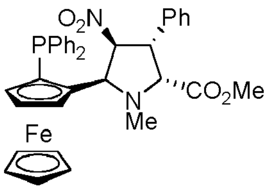

(D-Vb)

Method C

Method C describes the procedure for obtaining compounds of general formula (L/D-Ic),

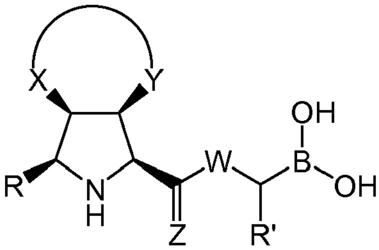

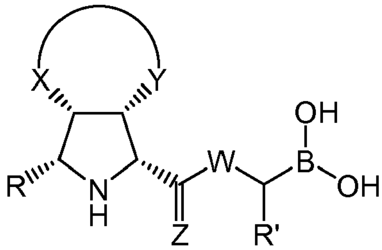

(L-Ic) (D-Ic)

wherein R, R', Z and W have the meaning given above, and X is linked to Y to form a cycloalkyl or heterocycloalkyl group,
which comprises:

a) reacting a (Z)-alkene of general formula (IIb),

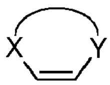

(IIb)

wherein residues X and Y are linked to each other thus forming a carbocycle or heterocycle;
with an imine of general formula (III) as described above;
in the presence of an organic or inorganic base, an aprotic solvent, a metallic salt, and an appropriate chiral catalyst, to afford compounds of formula (L/D-IVc):

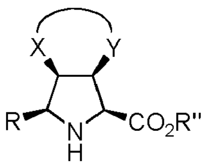

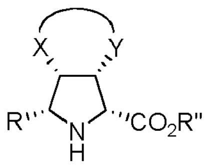

(L-IVc) (D-IVc)

b) reacting the compounds of formula (L-IVc) and (D-IVc) in a mixture selected from:

- a polar organic solvent and an aqueous solution of a hydroxide of an alkali or an alkaline earth metal;
- an appropriate organic solvent and a trialkyltin hydroxide;
- an organic acid and an organic solvent,
  to afford compounds of formula (L-VIc) and (D-VIc):

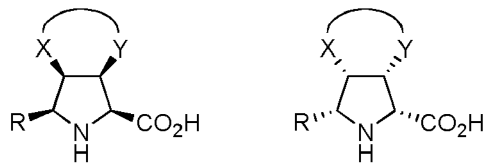

(L-VIc) (D-VIc)

wherein R, X and Y have the meaning given above;

c) reacting the compounds of formula (L/D-VIc) with a boronic ester of general formula (VII) as described above, or its hydrochloride salt;
in the presence of an organic base, a suitable coupling reagent, and an appropriate organic solvent, to afford an intermediate boronic ester;

d) deprotecting said intermediate boronic ester by treatment with isobutylboronic acid and an aqueous solution of hydrochloric acid to afford compounds of formula (L/D-Ic), wherein Z is O; and

e) reacting the compound of formula (I) obtained in step d) with a Lawesson reactive or a derivative thereof to afford the compound of formula (L/D-Ic) wherein Z is S.

In a particular embodiment, the chiral catalyst used to obtain compounds (L-IVc) is a compound of formula (D-Va) as defined above. In another particular embodiment, the chiral catalyst used to obtain compounds (D-IVc) is compounds of formula (L-Va) as defined above.

In a preferred embodiment of any of the Methods A, B and C falling within the process of the invention, Z is O and W is -NH- and, therefore, step e) is not performed.

The solvates, salts or prodrugs of the compounds of formula (D/L-I a-c) can be prepared following the procedures mentioned above.

<u>Use of the compounds of the invention</u>

According to a particular embodiment, the compounds of general formula (L/D-Ia-c) are useful for the treatment of various types of cancer, hematological malignancy, proliferative diseases, autoimmune diseases, neurodegenerative diseases or viral infections, by means of the inhibition of the active sites of the 20S core particle of the ubiquitin-proteasome system.

According to a particular embodiment, the cancer is selected from multiple myeloma, mantle cell lymphoma, pancreatic cancer, ovarian cancer, melanoma, hematologic cancers, renal cancer, osteosarcoma, fibrosarcoma, brain cancer, cervical cancer, epidermoid cancer, breast cancer, prostate cancer, colorectal cancer, lung cancer and liver cancer.

According to an embodiment of the invention, the autoimmune diseases can be inflammatory bowel disease, multiple sclerosis and organ-specific autoimmune diseases.

According to an additional embodiment of the invention, neurodegenerative diseases can be Alzheimer or prion protein-related conditions.

According to another embodiment of the invention, viral infections can be influenza A or herpes.

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

The compounds used in the present invention may be used with at least one other drug to provide a combination therapy. The at least other drug may be part of the composition, or be provided as a separate composition for administration at the same time or at different time.

Pharmaceutical compositions

Another aspect of the present invention refers to a pharmaceutical composition which comprises the compounds of formula (I) of the invention, or a pharmaceutically acceptable solvate or salt or prodrug thereof, and at least a pharmaceutically acceptable excipient.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil and similar. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition.

Examples of pharmaceutical compositions include any solid composition (tablets, pills, capsules, granules, etc.) or liquid composition (solutions, suspensions or emulsions) for oral, topical or parenteral administration.

In a preferred embodiment the pharmaceutical compositions are in oral delivery form. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate.

Solid oral compositions can be prepared by conventional methods of blending, filling or preparation of tablets. Repeated blending operations can be used to distribute the active ingredient in all the compositions that use large amounts of fillers. Such operations are conventional in the art. The tablets can be prepared, for example, by dry or wet granulation and optionally can be coated by well known methods in normal pharmaceutical practice, in particular using an enteric coating.

Pharmaceutical compositions can also be adapted for parenteral administration, such as sterile solutions, suspensions or lyophilized products in the appropriate unit dosage form. Suitable excipients such as fillers, buffering agents or surfactants can be used.

The mentioned formulations will be prepared using standard methods such as those described or referred to in the Spanish and U.S. Pharmacopoeias and similar reference texts.

In order to facilitate the understanding of the preceding ideas, some examples of experimental procedures and embodiments of the present invention are described below. These examples are merely illustrative.

**EXAMPLES**

**General Synthesis Methods**

The following general procedure describes the preparation of the exemplified compounds which correspond to the compounds of formula L/D-Ia, L/D-Ib and L/D-Ic in which Z is O and W is -NH-.

Method A

*A.1) Synthesis of compounds* (L/D-IVa)

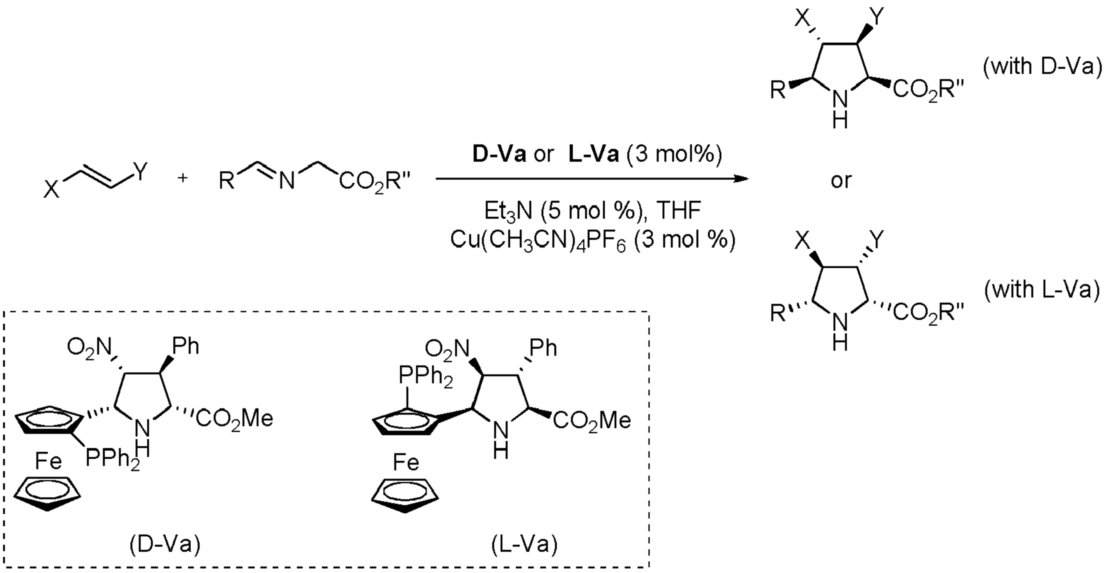


(D-Va) (L-Va)

A solution of the corresponding chiral catalyst (D-Va) or (L-Va) (0.015 mmol) and $Cu(CH_3CN)_4PF_6$ (5.2 mg, 0.014 mmol) in 1.0 ml of dry THF was stirred at -20 °C for 15 minutes. Then, a solution of the corresponding imine (III) (0.45 mmol) in 1.0 ml of an organic solvent (THF, toluene, dichloromethane, chloroform or 1,4-dioxane), triethylamine (3.2 μl, 0.023 mmol) and the corresponding alquene (IIa) (0.50 mmol) in 1.0 ml of an organic solvent solvent (THF, toluene, dichloromethane, chloroform or 1,4-dioxane) were successively added. The reaction was monitorized by TLC. Upon completion of the reaction, the mixture was filtered through a celite pad and the filtrate was concentrated under reduced pressure. The residue was purified by flash chromatography on silica gel (ethyl acetate:hexanes 1:2) to yield the corresponding cycloadduct (IVa). The enantiomeric excess was determined by HPLC, by comparison with the chromatogram recorded for the racemic mixture of the corresponding cycloadduct.

*A.2) Synthesis of compounds* (L/D-VIa)

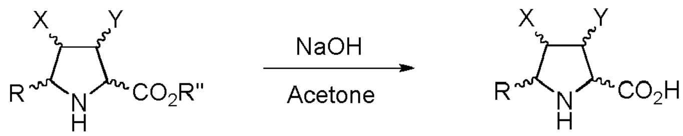


To a solution of the corresponding compound (IVa) (1.0 mmol) in acetone (3 ml) stirred at room temperature was added a solution of sodium hydroxide (88 mg, 2.2 mmol) in water (3 ml). The reaction mixture was stirred for 16 hours. Then, the solution was cooled to 0°C and acidified with HCl 2N to pH ≅ 2. A solid precipitated from the solution. This solid was filtered, washed with water and dried under vacuum to afford the corresponding product (VIa).

*A.3) Synthesis of compounds* (L/D-Ia)

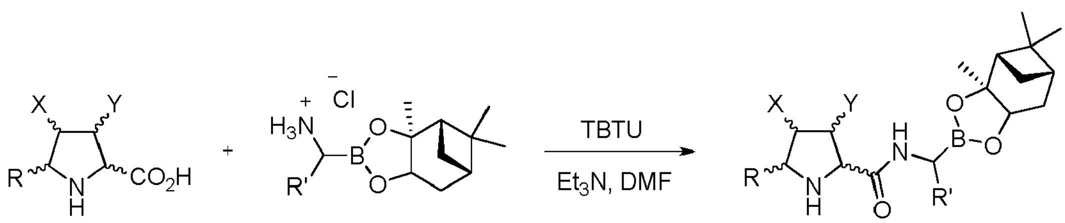


A mixture of the corresponding compound (VIa) (1 mmol), the corresponding aminoboronate hydrochloride

(VII) (301 mg, 1.0 mmol), O-(Benzotriazol-1-yl)-*N,N,N',N'*-tetramethyluronium tetrafluoroborate (353 mg, 1.1 mmol) in dry DMF (1.8 ml) under argon atmosphere was cooled to 0°C. Then, a mixture of triethylamine (0.42 ml, 3.0 mmol) and dry DMF (1 ml) was added dropwise, and the resulting mixture was stirred for 1.5 hours at 0°C. Then, ethyl acetate was added, and the organic layer was washed with water, dried over sodium sulphate, filtered and evaporated under reduced pressure.

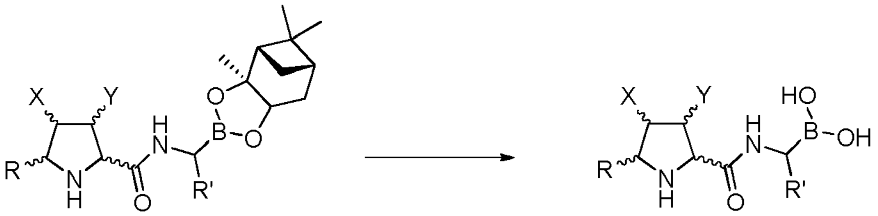

The crude mixture obtained in the previous step was dissolved in methanol (7 ml), hexane was added (7 ml), followed by isobutyl boronic acid (256 mg, 2.5 mmol) and HCl 2N (2 ml). The resulting mixture was stirred at room temperature for 16 hours. Then, the layers were separated and the methanolic layer was concentrated under reduced pressure. The resulting mixture was dissolved in ethyl acetate, washed with saturated aqueous sodium hydrogen carbonate, dried over sodium sulphate, filtered and evaporated under reduced pressure. The crude thus obtained was purified by precipitation, to afford the corresponding product (I'a).

Method B

*B.1) Synthesis of compounds* (L/D-IVb)

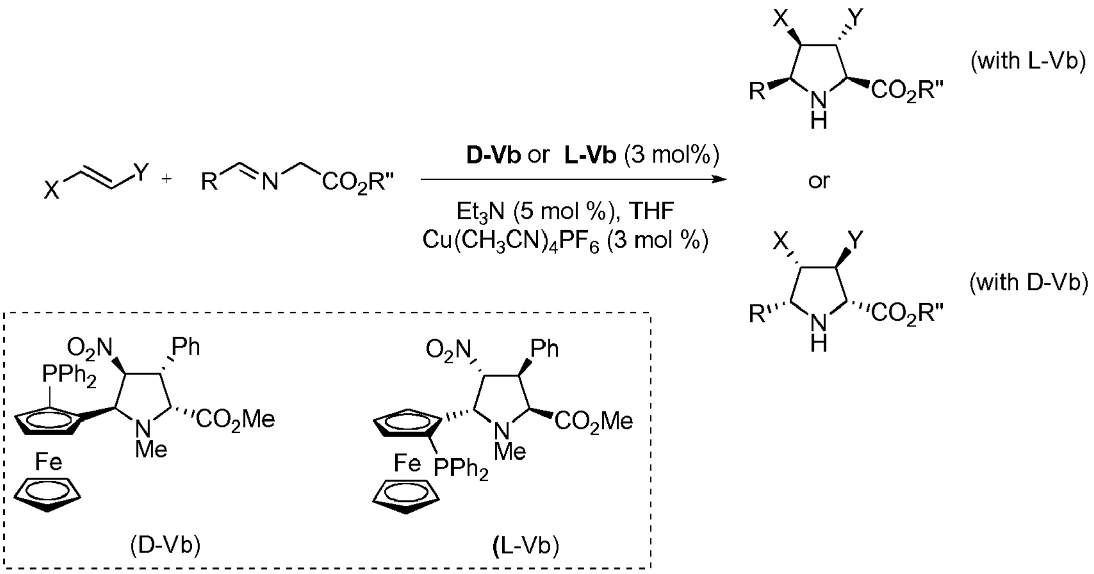

(D-Vb) (L-Vb)

A solution of the corresponding chiral catalyst (D-Vb) or (L-Vb) (0.015 mmol) and $Cu(CH_3CN)_4PF_6$ (5.2 mg, 0.014 mmol) in 1.0 ml of dry THF was stirred at -60 °C for 15 minutes. Then, a solution of the corresponding imine (III) (0.45 mmol) in 1.0 ml of an organic solvent (THF, toluene, dichloromethane, chloroform or 1,4-dioxane), triethylamine (3.2 µl, 0.023 mmol) and the corresponding alquene (IIa) (0.50 mmol) in 1.0 ml of an organic solvent solvent (THF, toluene, dichloromethane, chloroform or 1,4-dioxane) were successively added. The reaction was monitorized by TLC. Upon completion of the reaction, the mixture was filtered through a celite pad and the filtrate was concentrated under reduced pressure. The residue was purified by flash chromatography on silica gel (ethyl acetate:hexanes 1:2) to yield the corresponding cycloadduct (IVb). The enantiomeric excess was determined by HPLC, by comparison with the chromatogram recorded for the racemic mixture of the corresponding cycloadduct.

*B.2) Synthesis of compounds* (L/D-VIb)

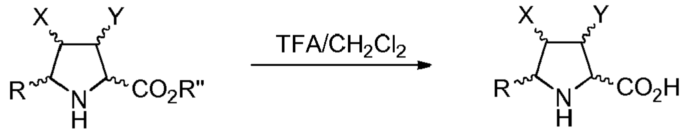


The corresponding compound (IVb) (1.0 mmol) was allowed to react with a trifluoroacetic acid/dichloromethane mixture (8ml:15ml). The resulting mixture was stirred at room temperature overnight, and the solvent was evaporated *in vacuo.* The crude obtained was purified by precipitation to afford the corresponding product (VIb).

*B.3) Synthesis of compounds* (L/D-Ib)

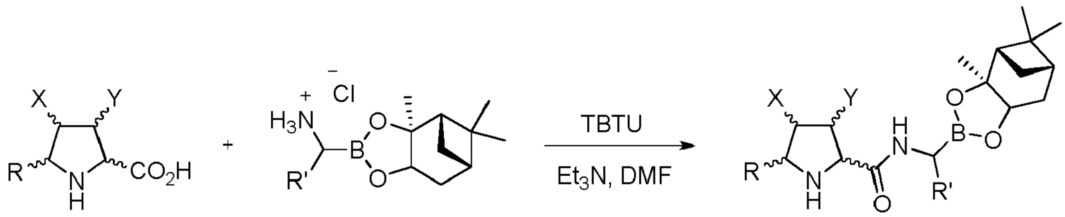


A mixture of the corresponding compound (VIb) (1 mmol), the corresponding aminoboronate hydrochloride (VII) (301 mg, 1.0 mmol), O-(Benzotriazol-1-yl)-*N,N,N',N'*-tetramethyluronium tetrafluoroborate (353 mg, 1.1 mmol) in dry DMF (1.8 ml) under argon atmosphere was cooled to 0 °C. Then, a mixture of triethylamine (0.42 ml, 3.0 mmol) and dry DMF (1 ml) was added dropwise, and the resulting mixture was stirred for 1.5 hours at 0 °C. Then, ethyl acetate was added, and the organic layer was washed with water, dried over sodium sulphate, filtered and evaporated under reduced pressure.

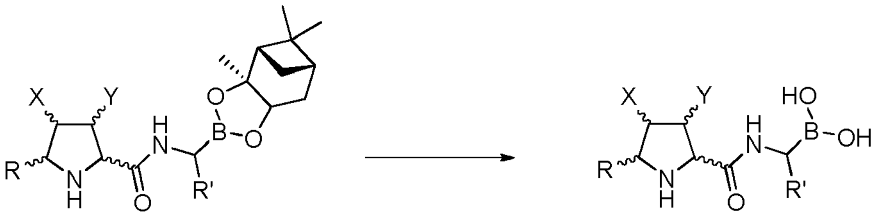

The crude mixture obtained in the previous step was dissolved in methanol (7 ml), hexane was added (7 ml), followed by isobutyl boronic acid (256 mg, 2.5 mmol) and HCl 2N (2 ml). The resulting mixture was stirred at room temperature for 16 hours. Then, the layers were separated and the methanolic layer was concentrated under reduced pressure. The resulting mixture was dissolved in ethyl acetate, washed with saturated aqueous sodium hydrogen carbonate, dried over sodium sulphate, filtered and evaporated under reduced pressure. The crude thus obtained was purified by precipitation, to afford the corresponding product (I'b).

<u>Method C</u>

*C.1) Synthesis of compounds* (L/D-IVc)

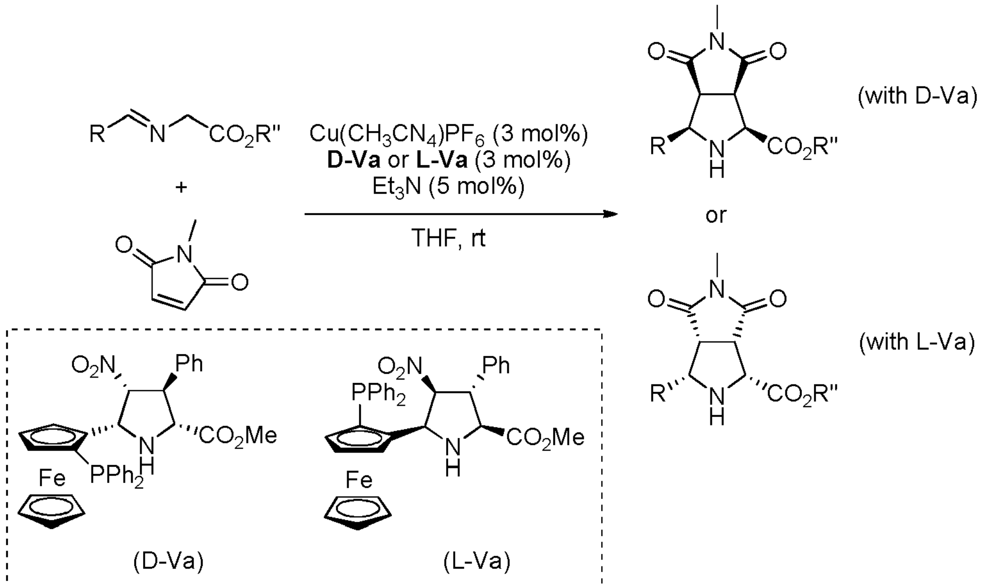


(with D-Va)

or

(with L-Va)

(D-Va) (L-Va)

A solution of the corresponding chiral catalyst (D-Va) or (L-Va) (0.015 mmol) and $Cu(CH_3CN)_4PF_6$ (5.2 mg, 0.014 mmol) in 1.0 mL of dry THF was stirred at 25 °C for 15 minutes. Then, solutions of the corresponding imine (III) (0.45 mmol) in 1.0 mL of an organic solvent (THF, toluene, dichloromethane, chloroform or 1,4-dioxane), triethylamine (3.2 μl, 0.023 mmol) and *N*-methyl maleimide (0.50 mmol) or the corresponding alquene (IIb) in 1.0 mL of an organic solvent solvent (THF, toluene, dichloromethane, chloroform or 1,4-dioxane) were successively added. The reaction was monitorized by TLC. Upon completion of the reaction, the mixture was filtered through a celite pad and the filtrate was concentrated under reduced pressure. The residue was purified by flash chromatography on silica gel (ethyl acetate:hexanes 1:2) to yield the corresponding cycloadduct (IVc). The enantiomeric excess was determined by HPLC, by comparison of the HPLC chromatogram recorded for the racemic mixture with the corresponding one of the enantiomerically enriched cycloadduct.

*C.2) Synthesis of compounds* (L/D-VIc)

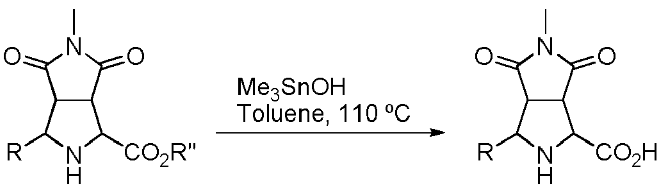


A solution of the corresponding compound (IVc) (1.0 mmol) and trimethyltin hydroxide in toluene (35 ml) was stirred at reflux for 19 hrs. Then, the solution was cooled to room temperature and acidified with HCl 1 N to pH ≅ 2. A solid precipitated from the solution. This solid was filtered, washed with water and dried under vacuum to afford the corresponding product (VIc).

*C.3) Synthesis of compounds* (L/D-Ic)

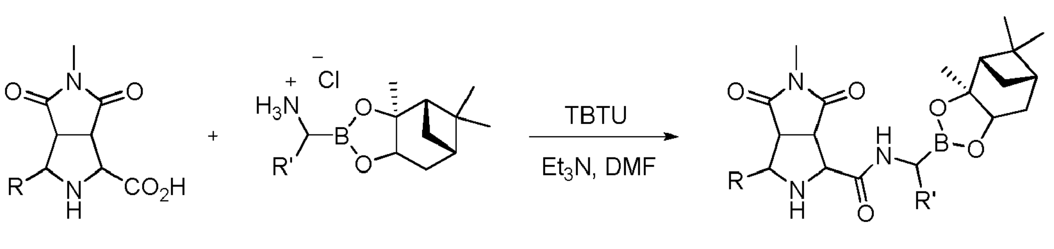

A mixture of the corresponding compound (VIc) (1 mmol), the corresponding aminoboronate hydrochloride (VII) (301 mg, 1.0 mmol), O-(Benzotriazol-1-yl)-*N,N,N',N'*-tetramethyluronium tetrafluoroborate (353 mg, 1.1 mmol) in dry DMF (1.8 ml) under argon atmosphere was cooled to 0 °C. Then, a mixture of triethylamine (0.42 ml, 3.0 mmol) and dry DMF (1 ml) was added dropwise, and the resulting mixture was stirred for 1.5 hours at 0 °C. Then, ethyl acetate was added, and the organic layer was washed with water, dried over sodium sulphate, filtered and evaporated under reduced pressure.

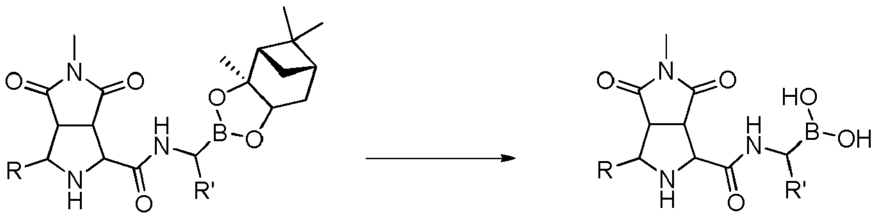

The crude mixture obtained in the previous step was dissolved in methanol (7 ml), hexane was added (7 ml), followed by isobutyl boronic acid (256 mg, 2.5 mmol) and HCl 2N (2 ml). The resulting mixture was stirred at room temperature for 16 hours. Then, the layers were separated and the methanolic layer was concentrated under reduced pressure. The resulting mixture was dissolved in ethyl acetate, washed with saturated aqueous sodium hydrogen carbonate, dried over sodium sulphate, filtered and evaporated under reduced pressure. The crude thus obtained was purified by precipitation, to afford the corresponding product (I'c).

**Synthesis of Compounds of the invention**

**Example 1:** *(R)-3-methyl-1-((2R,3R,4S,5R)-4-nitro-3,5-diphenylpyrrolidine-2-carboxamido)butylboronic acid, with the following structural formula:*

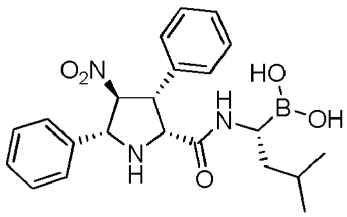

This compound was prepared following procedures described in Method A. White solid; $\nu_{max}/cm^{-1}$ 2951, 1550, 1390, 1364, 697; $^{1}H$ NMR (δ ppm, 500 MHz, $CD_3OD$) 7.64 (d, *J* = 7.3, 2H), 7.42 (t, *J* = 7.3, 2H), 7.38 (d, *J* = 7.3, 1 H), 7.37 - 7.28 (m, 5H), 5.25 (t, *J* = 7.7, 1 H), 4.83 (d, *J* = 7.6, 1 H), 4.63 (d, *J* = 8.9, 1 H), 4.47 (t, *J* = 8.3, 1 H), 2.42 (t, *J* = 7.5, 1 H), 1.50 - 1.03 (m, 1 H), 0.97 - 0.93 (m, 2H), 0.84 (d, *J* = 6.5, 3H), 0.82 (d, *J* = 6.5, 3H); $^{13}C$ NMR (δ ppm, 126 MHz, $CD_3OD$) 177.0, 140.2, 137.2, 129.9, 129.8, 129.7, 129.3, 128.2, 96.9, 68.2, 62.2, 54.1, 40.5, 26.8, 23.6, 22.4;

$[\alpha]_D^{25}$ = _ 82.6 (*c* 0.72, $CH_3OH$); MS (ESI, m/z): 424.29 $[M-1]^-$.

**Example 2:** *Preparation of (R)-3-methyl-1-((2S,3S,4R,5S)-4-nitro-3,5-diphenylpyrrolidine-2-carboxamido)butylboronic acid, with the following structural formula:*

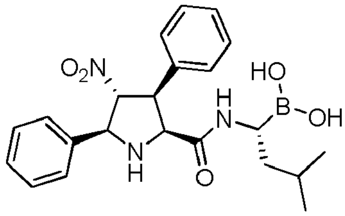

This compound was prepared following procedures described in Method A. White solid; $\nu_{max}/cm^{-1}$ 2953, 1550, 1385, 1365, 697; $^1H$ NMR (δ ppm, 500 MHz, $CD_3OD$) 7.64 (d, *J* = 7.1 Hz, 2H), 7.43 - 7.28 (m, 8H), 5.34 (t, *J* = 8.7 Hz, 1 H), 4.84 (d, *J* = 8.4 Hz, 1 H), 4.63 (d, *J* = 9.3 Hz, 1 H), 4.53 (t, *J* = 9.1 Hz, 1 H), 2.43 (dd, *J* = 9.0, 6.3 Hz, 1 H), 1.35 - 1.25 (m, 1 H), 1.06 - 0.97 (m, 1 H), 0.92 - 0.85 (m, 1 H), 0.81 (d, *J* = 6.5 Hz, 3H), 0.78 (d, *J* = 6.5 Hz, 3H); $^{13}C$ NMR (δ ppm, 126 MHz, $CD_3OD$) 177.6, 140.4, 136.4, 130.0, 129.8, 129.7, 129.6, 129.4, 128.3, 96.2, 68.2, 61.8, 54.4, 40.5, 26.7, 23.7, 22.2; $[\alpha]_D^{25}$ = +41.5 (*c* 0.35, $CH_3OH$). MS (ESI, m/z): 424.29 $[M-1]^-$.

**Example 3**: *Preparation of (R)-3-methyl-1-((2R,3S,4R,5R)-4-nitro-3,5-diphenylpyrrolidine-2-carboxamido)butylboronic acid, with the following structural formula:*

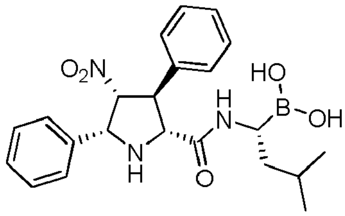

This compound was prepared following procedures described in Method B. White solid; $\nu_{max}/cm^{-1}$ 3115, 1546, 1363, 705; $^1H$ NMR (δ ppm, 500 MHz, $CD_3OD$) 7.51 (d, *J* = 7.0 Hz, 2H), 7.46 - 7.39 (m, 4H), 7.37 - 7.28 (m, 4H), 5.41 (dd, *J* = 7.3, 4.1 Hz, 1 H), 5.18 (d, *J* = 7.3 Hz, 1 H), 4.32 - 4.25 (m, 2H), 2.82 (t, *J* = 7.3 Hz, 1 H), 1.76 - 1.69 (m, 1 H), 1.54 - 1.42 (m, 2H), 0.96 (d, *J* = 6.6 Hz, 6H); $^{13}C$ NMR (δ ppm, 126 MHz, $CD_3OD$) 178.9, 139.8, 137.9, 130.3, 129.4, 129.3, 129.1, 129.0, 128.4, 97.0, 67.0, 64.6, 55.1, 41.0, 27.1, 23.7, 22.7; $[\alpha]_D^{25}$ = - 56.4 (*c* 0.66, $CH_3OH$); MS (ESI, m/z): 424.08 $[M-1]^-$.

**Example 4:** *Preparation of (R)-3-methyl-1-((2S,3R,4S,5S)-4-nitro-3,5-diphenylpyrrolidine-2-carboxamido)butylboronic acid, with the following structural formula:*

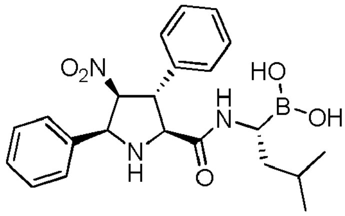

This compound was prepared following procedures described in Method B. White solid; $\nu_{max}/cm^{-1}$ 2952, 1551, 1365, 697; $^1H$ NMR (δ ppm, 500 MHz, $CD_3OD$) 7.52 (d, *J* = 7.2 Hz, 2H), 7.42 - 7.40 (m, 4H), 7.38 - 7.27 (m, 4H), 5.47 (dd, *J* = 7.4, 4.3 Hz, 1 H), 5.13 (d, *J* = 7.4 Hz, 1 H), 4.26 - 4.21 (m, 2H), 2.83 (t, *J* = 7.8 Hz, 1 H), 1.62 - 1.56 (m, 1 H), 1.36 (t, *J* = 7.3 Hz, 2H), 0.91 (d, *J* = 6.5 Hz, 3H), 0.89 (d, *J* = 6.5 Hz, 3H); $^{13}C$ NMR (δ ppm, 126 MHz, $CD_3OD$) 178.1,

139.3, 137.7, 130.2, 129.4, 129.3, 129.2, 129.1, 128.4, 97.1, 67.1, 65.0, 55.6, 41.0, 27.0, 23.6, 22.7; $[\alpha]_D^{25}$ = - 75.2 (*c* 0.15, $CH_3OH$); MS (ESI, m/z): 424.08 [M-1]$^-$.

**Example 5:** *Preparation of (R)-3-methyl-1-((2R,3R,4S,5R)-4-nitro-5-phenyl-3-p-tolylpyrrolidine-2-carboxamido)butylboronic acid, with the following structural formula:*

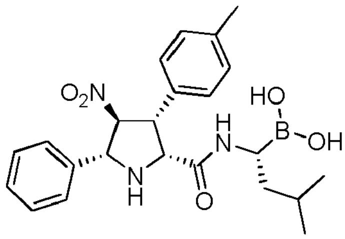

This compound was prepared following procedures described in Method A. White solid; $\nu_{max}/cm^{-1}$ 2955, 1556, 1391, 1351, 527; $^1H$ NMR (δ ppm, 500 MHz, $CD_3OD$) 7.86 - 7.85 (m, 2H), 7.63 - 7.55 (m, 3H), 7.34 (d, *J* = 8.0 Hz, 2H), 7.26 (d, *J* = 7.7 Hz, 2H), 6.23 (t, *J* = 10.5 Hz, 1 H), 5.32 (d, *J* = 10.1 Hz, 1 H), 4.83 (d, *J* = 9.3 Hz, 1H), 4.68 (d, *J* = 10.6 Hz, 1 H), 3.04 (t, *J* = 6.5 Hz, 1 H), 2.38 (s, 3H), 1.06 - 1.04 (m, 2H), 0.90 - 0.84 (m, 1 H), 0.76 (d, *J* = 6.5 Hz, 3H), 0.71 (d, *J* = 6.3 Hz, 3H). $^{13}C$ NMR (δ ppm, 126 MHz, $CD_3OD$) 167.0, 140.3, 132.1, 130.9, 130.7, 130.1, 129.5, 90.1, 66.0, 63.1, 52.7, 40.0, 26.1, 23.5, 22.4, 21.3; $[\alpha]_D^{25}$ = - 4.4 (*c* 0.30, $CH_3OH$); MS (ESI, m/z): 438.02 [M-1]$^-$.

**Example 6:** *Preparation of (R)-1-((2R,3R,4S,5R)-3-(4-fluorophenyl)-4-nitro-5-phenylpyrrolidine-2-carboxamido)-3-methylbutylboronic acid, with the following structural formula:*

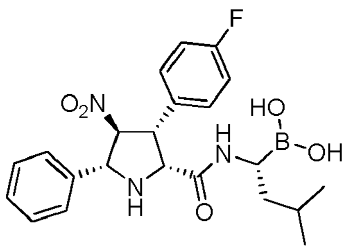

This compound was prepared following procedures described in Method A. White solid; $\nu_{max}/cm^{-1}$ 2953, 1547, 1422, 1379, 699, 531; $^1H$ NMR (δ ppm, 500 MHz, $CD_3OD$) 7.64 (d, *J* = 7.2 Hz, 2H), 7.43 (t, *J* = 7.3 Hz, 2H), 7.40 - 7.33 (m, 3H) 7.06 (t, *J* = 8.7 Hz, 2H), 5.21 (t, *J* = 7.4 Hz, 1 H), 4.60 (d, *J* = 8.8 Hz, 1 H), 4.47 (t, *J* = 7.6 Hz, 1 H), 2.42 (t, *J* = 7.7 Hz, 1 H), 1.50 - 1.45 (m, 1 H), 1.07 - 1.01 (m, 1 H), 0.98 - 0.92 (m, 1 H), 0.86 (d, *J* = 6.6 Hz, 3H), 0.84 (d, *J* = 6.6 Hz, 3H); $^{13}C$ NMR (δ ppm, 126 MHz, $CD_3OD$) 176.8, 164.0 (d, $^1J_{C-F}$ = 245.9 Hz), 140.2, 133.3 (d, $^4J_{J-F}$ = 2.7 Hz), 131.8 (d, $^3J_{C-F}$ = 8.2 Hz), 129.9, 129.8, 128.2, 116.6 (d, $^2J_{C-F}$ = 21.7 Hz), 96.9, 68.1, 62.3, 53.4, 40.7, 26.7, 23.6, 22.4. $[\alpha]_D^{25}$ = -62.7 (*c* 0.45, $CH_3OH$); MS (ESI, m/z): 442.02 [M-1]$^-$.

**Example 7:** *Preparation of (R)-1-((2R,3R,4S,5R)-3-(4-methoxyphenyl)-4-nitro-5-phenylpyrrolidine-2-carboxamido)-3-methylbutylboronic acid, with the following structural formula:*

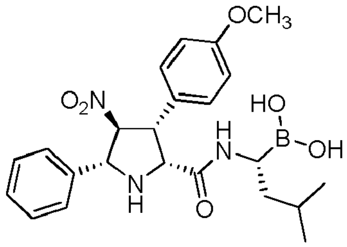

This compound was prepared following procedures described in Method A. White solid; $^1$H NMR (δ ppm, 500 MHz, $CD_3OD$) 7.64 (d, *J* = 7.5 Hz, 2H), 7.42 (t, *J* = 7.2 Hz, 2H), 7.38 (d, *J* = 6.9 Hz, 1 H), 7.23 (d, *J* = 8.8 Hz, 2H), 6.86 (d, *J* = 8.0 Hz, 2H), 5.19 (t, *J* = 7.7 Hz, 1 H), 4.58 (d, *J* = 8.1 Hz, 1 H), 4.39 (t, *J* = 8.0 Hz, 1 H), 2.45 (t, *J* = 7.8 Hz, 1 H), 1.50 - 1.44(m, 1 H), 1.06 - 0.94 (m, 2H), 0.85 (d, *J* = 7.0 Hz, 3H), 0.83 (d, *J* = 6.9 Hz, 3H); MS (ESI, m/z): 454.33 [M-1]$^-$.

**Example 8:** *Preparation of (R)-3-methyl-1-((2R,3R,4S,5R)-4-nitro-3-(4-nitrophenyl)-5-phenylpyrrolidine-2-carboxamido)butylboronic acid, with the following structural formula:*

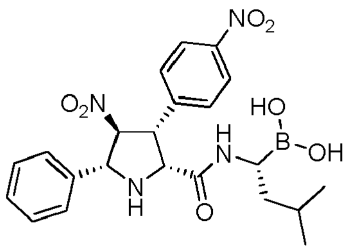

This compound was prepared following procedures described in Method A. White solid; $^{13}$C NMR (δ ppm, 101 MHz, $CD_3OD$) 171.0, 149.0, 145.0, 131.1, 131.1, 130.0, 129.9, 128.3, 124.7, 96.1, 68.1, 62.4, 53.6, 40.9, 26.6, 23.5, 22.6, 22.4; MS (ESI, m/z): 469.09 [M-1]$^-$.

**Example 9**: *Preparation of (R)-3-methyl-1-((2R,3R,4S,5R)-3-(naphthalen-2-yl)-4-nitro-5-phenylpyrrolidine-2-carboxamido)butylboronic acid, with the following structural formula:*

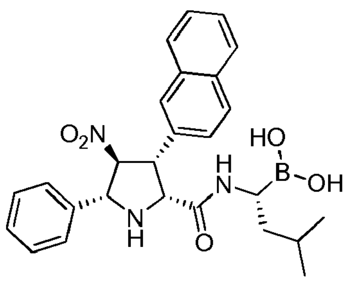

This compound was prepared following procedures described in Method A. White solid; $^{13}$C NMR (δ ppm, 101 MHz, $CD_3OD$) 176.7, 134.6, 134.4, 129.9, 129.8, 129.6, 129.0, 128.8, 128.7, 128.3, 127.5, 127.4, 96.7, 68.1, 62.4, 54.2, 40.4, 26.4, 23.4, 22.3; MS (ESI, m/z): 474.00 [M-1]$^-$.

**Example 10**: *Preparation of (R)-3-methyl-1-((2R,3R,4S,5R)-4-nitro-5-phenyl-3-(thiophen-2-yl)pyrrolidine-2-carboxamido)butylboronic acid, with the following structural formula:*

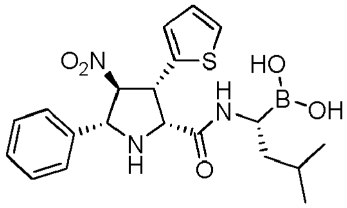

This compound was prepared following procedures described in Method A. White solid; $\nu_{max}/cm^{-1}$ 2953, 1556, 1384, 1351, 711; $^1H$ NMR (δ ppm, 500 MHz, $CD_3OD$) 7.65 (d, *J* = 7.2 Hz, 2H), 7.42 (t, *J* = 7.3 Hz, 2H), 7.38 (d, *J* = 7.2 Hz, 1 H), 7.34 (d, *J* = 4.8 Hz, 1 H), 7.04 (d, *J* = 3.0 Hz, 1 H), 6.99 - 6.95 (m, 1 H), 5.16 (t, *J* = 6.8 Hz, 1 H), 4.81 - 4.77(m, 2H), 4.59 (d, *J* = 8.0 Hz, 2H), 2.52 (t, *J* = 7.2 Hz, 1 H), 1.57 - 1.50 (m, 2H), 0.87 (t, *J* = 7.1 Hz, 6H); $^{13}C$ NMR (δ ppm, 126 MHz, $CD_3OD$) 176.6, 140.3, 138.9, 129.8, 129.7, 128.6, 128.3, 128.0, 126.9, 98.0, 68.2, 62.5, 40.7, 26.8, 23.7, 22.4; $[\alpha]_D^{25}$ = - 26.6 (*c* 0.65, $CH_3OH$); MS (ESI, m/z): 430.09 $[M-1]^-$.

**Example 11:** *Preparation of (R)-1-((2R,3R,4S,5R)-3-(furan-2-yl)-4-nitro-5-phenylpyrrolidine-2-carboxamido)-3-methylbutylboronic acid, with the following structural formula:*

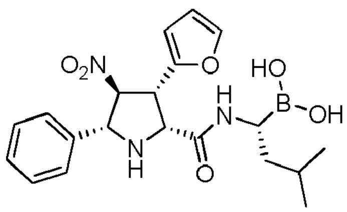

This compound was prepared following procedures described in Method A. White solid; $\nu_{max}/cm^{-1}$ (KBr) 3430, 1723, 1633, 1511, 1226. $^1H$ NMR (δ ppm, 500 MHz, $CD_3OD$) 7.60 (d, *J* = 7.3 Hz, 2H), 7.46 (d, *J* = 3.8 Hz, 1 H), 7.43 - 7.34 (m, 3H), 6.35 (s, 1 H), 6.32 (s, 1 H), 5.25 (t, *J* = 7.8 Hz, 1 H), 4.76 (d, *J* = 7.7 Hz, 1 H), 4.65 - 4.55 (m, 2H), 2.59 (t, *J* = 7.5 Hz, 1 H), 1.59 - 1.48 (m, 1 H), 1.22 (7, *J* = 7.0 Hz, 2H), 0.91 - 0.88(m, 6H); $^{13}C$ NMR (δ ppm, 126MHz, $CD_3OD$) 167.0, 145.3, 132.0, 130.6, 130.1, 112.0, 111.6, 89.9, 65.7, 61.8, 46.7, 40.4, 26.4, 23.5, 22.5.; $[\alpha]_D^{25}$ = _ 17.6 (*c* 0.45, $CH_3OH$); MS (ESI, m/z): 414.08 $[M-1]^-$.

**Example 12:** *Preparation of (R)-3-methyl-1-((2R,3R,4S,5R)-5-(naphthalen-2-yl)-4-nitro-3-phenylpyrrolidine-2-carboxamido)butylboronic acid, with the following structural formula:*

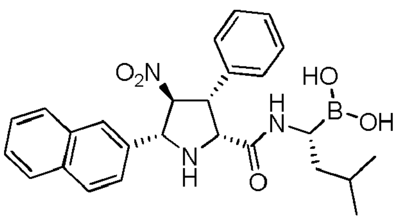

This compound was prepared following procedures described in Method A. White solid; $\nu_{max}/cm^{-1}$ 1556, 1383, 1366, 746; $^1H$ NMR (δ ppm, 500 MHz, $CD_3OD$) 8.10 (s, 1H), 7.95 (d, *J* = 8.5 Hz, 1 H), 7.91 - 7.88 (m, 2H), 7.81 (d, *J* = 9.2 Hz, 1 H), 7.53 - 7.51 (m, 2H), 7.38 - 7.29 (m, 5H), 5.41 (t, *J* = 8.2 Hz, 1 H), 5.03 (d, *J* = 8.3 Hz, 1 H), 4.70 (d, *J* = 9.3 Hz, 1 H), 4.55 (t, *J* = 7.4 Hz, 1 H), 2.47 - 2.44 (m, 1 H), 1.53 - 1.46 (m, 1 H), 1.09 - 0.98 (m, 2H), 0.86 (d, J = 6.4 Hz,

3H), 0.83 (d, J = 6.3 Hz, 3H); $^{13}C$ NMR (δ ppm, 126 MHz, $CD_3OD$) 167.1, 135.7, 134.5, 132.2, 130.8, 130.3, 129.6, 129.5, 128.9, 128.8, 128.2, 126.0, 89.9, 66.2, 63.1, 52.9, 40.1, 26.1, 23.5, 22.5; $[\alpha]_D^{25}$ = -28.3 (*c* 0.30, $CH_3OH$); MS (ESI, m/z): 474.31 $[M-1]^-$.

**Example 13:** *Preparation of (R)-3-methyl-1-((2R,3R,4S,5R)-5-(naphthalen-1-yl)-4-nitro-3-phenylpyrrolidine-2-carboxamido)butylboronic acid, with the following structural formula:*

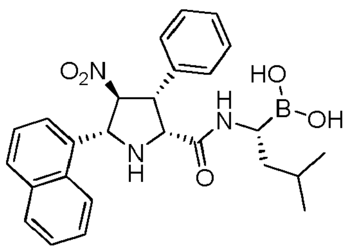

This compound was prepared following procedures described in Method A. White solid; $\nu_{max}/cm^{-1}$ 1544, 1368, 697; $^1H$ NMR (δ ppm, 500 MHz, $CD_3OD$) 8.28 (d, *J* = 7.3 Hz, 1 H), 8.11 (d, *J* = 9.0 Hz, 1 H), 7.94 - 7.91 (m, 2H), 7.62 (t, *J* = 7.6 Hz, 1 H), 7.57 - 7.50 (m, 2H), 7.42 - 7.30 (m, 5H), 5.79 (d, *J* = 8.0 Hz, 1 H), 5.48 (t, *J* = 7.9 Hz, 1 H), 4.76 (d, *J* = 9.2 Hz, 1 H), 4.58 (t, *J* = 8.1 Hz, 1 H), 2.48 - 2.40 (m, 1 H), 1.56 - 1.46 (m, 1 H), 1.11 - 0.97 (m, 2H), 0.86 (d, *J* = 6.5 Hz, 3H), 0.83 (d, *J* = 6.5 Hz, 3H); $^{13}C$ NMR (δ ppm, 126 MHz, $CD_3OD$) 168.5, 130.2, 129.9, 129.8, 129.7, 129.3, 129.2, 127.8, 127.6, 126.9, 126.7, 125.8, 123.5, 89.0, 65.2, 63.4, 54.8, 40.5, 26.8, 23.6, 22.4; $[\alpha]_D^{25}$ = - 29.1 (*c* 0.45, $CH_3OH$); MS (ESI, m/z): 474.08 $[M-1]^-$.

**Example 14:** *Preparation of (R)-1-((2R,3R,4S,5S)-5-(furan-2-yl)-4-nitro-3-phenylpyrrolidine-2-carboxamido)-3-methylbutylboronic acid, with the following structural formula:*

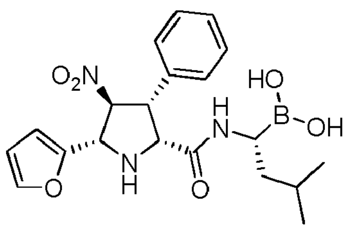

This compound was prepared following procedures described in Method A. White solid; $\nu_{max}/cm^{-1}$ 1555, 1383, 1348, 748; $^1H$ NMR (δ ppm, 500 MHz, $CD_3OD$) 7.76 (s, 1 H), 7.45 - 7.41 (m, 5H), 6.99 (d, *J* = 2.8 Hz, 1 H), 6.60 (s, 1 H), 6.30 (t, *J* = 10.2 Hz, 1 H), 5.61 (d, *J* = 9.9 Hz, 1 H), 4.69 (t, *J* = 9.9 Hz, 1 H), 2.95 - 2.90 (m, 1 H), 1.04 - 1.02 (m, 2H), 0.92 - 0.84(m, 1 H), 0.72 (d, *J* = 5.6 Hz, 3H), 0.65 (d, *J* = 4.8 Hz, 3H); $^{13}C$ NMR (δ ppm, 126MHz, $CD_3OD$) 199.1, 166.1, 146.9, 144.8, 132.4, 130.2, 129.6, 114.6, 112.5, 88.1, 63.2, 58.5, 52.4, 40.0, 26.0, 23.4, 22.4; $[\alpha]_D^{25}$ = _ 30.9 (*c* 0.60, $CH_3OH$); MS (ESI, m/z): 414.08 $[M-1]^-$.

**Example 15**: *Preparation of (R)-3-methyl-1-((2R,3R,4S,5R)-4-nitro-3-phenyl-5-(pyridin-3-yl)pyrrolidine-2-carboxamido)butylboronic acid, with the following structural formula:*

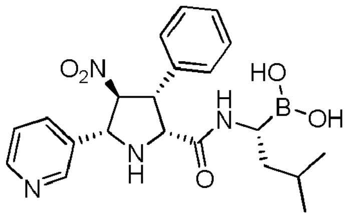

This compound was prepared following procedures described in Method A. White solid; $^1$H NMR (δ ppm, 500 MHz, $CD_3OD$) 8.77 (d, *J* = 8.2 Hz, 1 H), 8.55 (t, *J* = 4.7 Hz, 1 H), 8.23 (t, *J* = 7.3 Hz, 1 H), 7.54 - 7.50 (m, 1 H), 7.36 - 7.29 (m, 5H), 5.38 (t, *J* = 7.9 Hz, 1 H), 4.94 (d, *J* = 8.2 Hz, 1 H), 4.65 (d, *J* = 9.0 Hz, 1 H), 4.56 (t, *J* = 8.4 Hz, 1 H), 2.39 (t, *J* = 8.3 Hz, 1 H), 1.52 - 1.47 (m, 1 H), 1.07 - 0.94 (m, 2H), 0.85 (d, *J* = 6.5 Hz, 3H), 0.82 (d, *J* = 6.8 Hz, 3H); MS (ESI, m/z): 425.20 $[M-1]^-$.

**Example 16**: *Preparation of (R)-3-methyl-1-((2R,3S,4R,5R)-5-(naphthalen-1-yl)-4-nitro-3-phenylpyrrolidine-2-carboxamido)butylboronic acid, with the following structural formula:*

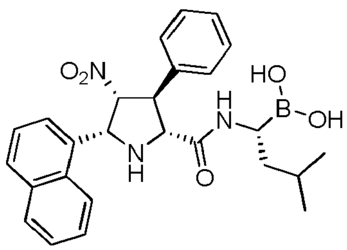

This compound was prepared following procedures described in Method B. White solid; $\nu_{max}/cm^{-1}$ 2953, 1551, 1386, 699; $^1$H NMR (δ ppm, 500 MHz, $CD_3OD$) 8.06 - 8.01 (m, 2H), 7.90 (d, *J* = 8.0 Hz, 1 H), 7.85 (d, *J* = 8.1 Hz, 1 H), 7.58 - 7.45 (m, 7H), 7.39 (t, *J* = 7.3 Hz, 1 H), 5.98 (d, *J* = 6.9 Hz, 1 H), 5.59 (dd, *J* = 6.9, 3.4 Hz, 1 H), 4.42 (d, *J* = 6.7 Hz, 1 H), 4.40 - 4.36 (m, 1 H), 2.85 (t, *J* = 7.4 Hz, 1 H), 1.80 - 1.75 (m, 1 H), 1.57 - 1.45 (m, 2H), 0.99 (d, *J* = 6.6 Hz, 6H); $^{13}$C NMR (δ ppm, 126MHz, $CD_3OD$) 179.2, 140.2, 135.0, 133.1, 132.4, 130.5, 130.0, 129.2, 129.0, 127.6, 126.7, 126.4, 126.0, 123.3, 96.2, 64.0, 63.2, 55.4, 41.1, 27.1, 23.7, 22.8; MS (ESI, m/z): 474.28 $[M-1]^-$.

**Example 17**: *Preparation of (R)-1-((2S,3S,4S,5R)-3,4-bis(methoxycarbonyl)-5-phenylpyrrolidine-2-carboxamido)-3-methylbutylboronic acid, with the following structural formula:*

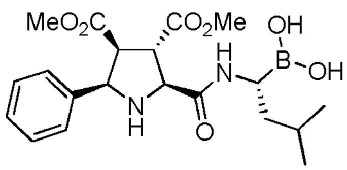

This compound was prepared following procedures described in Method B. White solid; $\nu_{max}/cm^{-1}$ 1731, 1435, 1199, 1170, 699; $^1$H NMR (δ ppm, 500 MHz, $CD_3OD$) 7.43 (d, *J* = 7.4 Hz, 2H), 7.33 (t, *J* = 7.4 Hz, 2H), 7.28 (d, *J* = 7.1 Hz, 1 H), 4.79 (d, *J* = 8.2 Hz, 1 H), 4.33 (d, *J* = 7.0 Hz, 1 H), 3.79 (s, 3H), 3.67 (t, *J* = 5.7 Hz, 1 H), 3.60 (t, *J* = 5.5 Hz, 1 H), 3.20 (s, 3H), 2.89 (t, *J* = 7.5 Hz, 1 H), 1.78 - 1.71 (m, 1 H), 1.45 (t, *J* = 7.3 Hz, 2H), 0.99 (s, 3H), 0.98 (s, 3H); $[\alpha]_D^{25}$ = + 23.6 (*c* 0.45, $CH_3OH$); MS (ESI, m/z): 419.25 $[M-1]^-$.

**Example 18:** *Preparation of (R)-3-methyl-1-((1S,3R,3aS,6aR)-5-methyl-4,6-dioxo-3-phenyloctahydropyrrolo[3,4-c]pyrrole-1-carboxamido)butylboronic acid, with the following structural formula:*

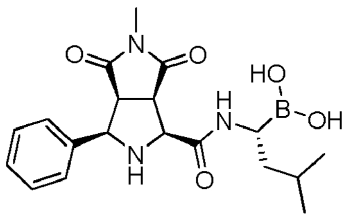

**This** compound was prepared following procedures described in Method C. White solid; $\nu_{max}/cm^{-1}$ 2953, 1698, 1382, 1285; $^1H$ NMR (δ ppm, 500 MHz, $CD_3OD$) 7.45 (d, *J* = 7.2 Hz, 2H), 7.35 (t, *J* = 7.3 Hz, 2H), 7.30 (t, *J* = 7.2 Hz, 1 H), 4.59 (d, *J* = 9.0 Hz, 1 H), 4.18 (d, *J* = 7.0 Hz, 1 H), 3.71 (t, *J* = 7.4 Hz, 1 H), 3.55 (t, *J* = 8.7 Hz, 1 H), 2.90 (t, *J* = 7.7 Hz, 1 H), 2.85 (s, 3H), 1.79 - 1.73 (m, 1 H), 1.49 (t, *J* = 6.5 Hz, 2H), 1.00 (d, *J* = 1.7 Hz, 3H), 0.98 (d, *J* = 1.7 Hz, 3H); $^{13}C$ NMR (δ ppm, 126MHz, $CD_3OD$) 177.1, 139.3, 129.1, 128.8, 128.5, 128.4, 65.0, 60.4, 50.5, 40.3, 27.2, 25.0, 23.3;

$[\alpha]_D^{25}$ = + 62.9 (*c* 0.30, $CH_3OH$); MS (ESI, m/z): 386.27 $[M-1]^-$.

**Example 19:** *Preparation of (R)-3-methyl-1-((1R,3S,3aR,6aS)-5-methyl-4,6-dioxo-3-phenyloctahydropyrrolo[3,4-c]pyrrole-1-carboxamido)butylboronic acid, with the following structural formula:*

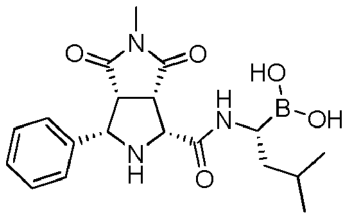

This compound was prepared following procedures described in Method C. White solid; $\nu_{max}/cm^{-1}$ 2953, 1701, 1381, 1285; $^1H$ NMR (δ ppm, 500 MHz, $CD_3OD$) 7.42 - 7.39 (m, 2H), 7.32 - 7.29 (m, 2H), 7.28 - 7.24 (m, 1 H), 4.55 (dd, *J* = 8.9, 2.8 Hz, 1 H), 4.14 (t, *J* = 6.2 Hz, 1 H), 3.67 (dd, *J* = 16.9, 7.4 Hz, 1 H), 3.53 - 3.48 (m, 1 H), 2.86 (t, *J* = 7.7 Hz, 1 H), 2.80 (s, 3H), 1.75 - 1.66 (m, 2H), 1.54 - 1.48 (m, 1 H), 0.97 (d, *J* = 6.7 Hz, 3H), 0.95 (d, *J* = 6.6 Hz, 3H); MS (ESI, m/z): 386.27 $[M-1]^-$.

<u>**Biological activity**</u>

Activity of the compounds described within this invention was compared with the *L*-proline *L*-leucine boronic acid MG-319 (IUPAC name: {(*R*)-3-methyl-1-[(S)-pyrrolidine-2-carboxamido]butyl}boronic acid, obtained in house) disclosed in WO 96/13266.

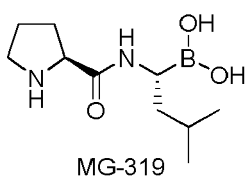

MG-319

**Example 20:** *In vitro enzymatic assay of proteasome inhibitory activity.*

***Components of Assay***

*Substrate:* Suc-Leu-Leu-Val-Tyr-AMC

*Assay buffer:* 250 mM HEPES, pH 7.5, 0.5 mM EDTA, 0.03% SDS, 0.05% Igepal CA-630.

*Enzyme:* Human erythrocyte 20S proteasome

***Assay procedure***

*In vitro* proteasome inhibitory activity of the compounds of the invention is evaluated by spectrofluorimetric quantitation of AMC. Stock solutions of compounds of the invention are prepared in dimethyl sulphoxide (DMSO). Test compound and/or vehicle is preincubated with 2.5 mg/ml enzyme (human erythrocyte 20S proteasome) in modified HEPES buffer pH 7.5 and 1% final concentration of DMSO for 15 minutes at 37°C. The reaction is initiated by addition of 10 μM Suc-Leu-Leu-Val-Tyr-AMC for another 30 minutes incubation period at 37°C. Determination of the release of AMC is read spectrophotometrically (excitation, 380 nm; emission, 460 nm). $IC_{50}$ values are determined by a non-linear, least squares regression analysis using MathIQTM (ID Business Solutions Ltd., UK). Lactacystin is used as positive control.

| Compound | Concentration (nM) | % Inhibition | $IC_{50}$ (nM) |
|---|---|---|---|
| Example 1 | 100 | 73 | 39 |
| Example 2 | 1000 | 70 | 430 |
| Example 4 | 50 | 51 | 46 |
| Example 5 | 500 | 77 | 78 |
| Example 6 | 50 | 70 | 17 |
| Example 8 | 500 | 69 | 150 |
| Example 9 | 500 | 69 | 150 |
| Example 10 | 50 | 79 | 12 |
| Example 11 | 50 | 81 | 7.55 |
| Example 12 | 50 | 50 | 46 |
| Example 13 | 50 | 54 | 39 |
| Example 15 | 500 | 72 | 100 |
| Example 16 | 500 | 33 | - |
| Example 17 | 500 | 20 | - |
| Example 18 | 1000 | 53 | 920 |
| Example 19 | 100 | 68 | 46 |
| MG-319 | 500 | 25 | - |
| Lactacystin | - | - | 110 |

**Example 21:** *Biological activity in cancer cell lines.*

Cell culture-based assays were used to evaluate the ability of compounds of the invention to inhibit cancer cell growth.

Cells were obtained from the American Type Culture Collection (ATCC) and the European Collection of Cell Cultures. Cells were thawed in their appropriate media plus supplements (see table below). Cells were passaged at confluence by washing once in HBSS cation-free followed by a 3 minutes incubation with trypsin ([0.5 μg/ml]/EDTA [0.2 μg/ml]) (Gibco-BRL, 15400054) solution in HBSS at 37°C (except non-adherent cell lines - hematologic), and transferred to their appropriate media plus supplements. Prior to seeding at defined cell concentration, cells were recovered in medium, centrifuged, taken up in medium and counted.

| Cell line | Source | Culture Medium | Cells/well |
|---|---|---|---|
| 143B | ECACC - 91112502 | EMEM + 2mM Glutamine + 1% Non-essential Aminoacids + 10 FCS | 10000 |
| A431 | ECACC - 85090402 | DEMEM High Glucose (4500 mg/l) + 10 FCS | 10000 |
| A549 | ATCC - CCL-185 | DEMEM High Glucose (4500 mg/l) + 10 FCS | 5000 |
| BxPC-3 | ECACC - 93120816 | DEMEM High Glucose (4500 mg/l) + 10 FCS | 10000 |
| CCRF-CEM | ECACC - 85112105 | RPMI + 2mM Glutamine + 10% FCS | 20000 |
| COLO 205 | ECACC - 87061208 | DEMEM High Glucose (4500 mg/l) + 10 FCS | 10000 |
| Daoy | ECACC - 58483215 | DEMEM High Glucose (4500 mg/l) + 10 FCS | 5000 |
| DU-145 | ATCC - HTB-81 | DEMEM High Glucose (4500 mg/l) + 10 FCS | 10000 |
| HCT-116 | ECACC - 91091005 | DEMEM High Glucose (4500 mg/l) + 10 FCS | 5000 |
| HeLa | ECACC - 93021013 | DEMEM High Glucose (4500 mg/l) + 10 FCS | 10000 |
| Hep-G2 | ECACC - 85011430 | EMEM + 2mM Glutamine + 1% Non-essential Aminoacids + 10 FCS | 10000 |
| HT-1080 | ECACC - 85111505 | DEMEM High Glucose (4500 mg/l) + 10 FCS | 10000 |
| HT-29 | ECACC - 91072201 | DEMEM High Glucose (4500 mg/l) + 10 FCS | 10000 |
| Jurkat | ECACC - 88042803 | DEMEM High Glucose (4500 mg/l) + 10 FCS | 10000 |
| K562 | ECACC - 89121407 | RPMI + 2mM Glutamine + 10% FCS | 20000 |
| LNCaP | ECACC - 89110211 | RPMI + 2mM Glutamine + 10% FCS | 10000 |
| LoVo | ATCC - CCL-229 | DEMEM High Glucose (4500 mg/l) + 10 FCS | 10000 |
| LS174T | ECACC - 87060401 | EMEM + 2mM Glutamine + 1% Non-essential Aminoacids + 10 FCS | 10000 |
| MCF-7 | ECACC - 86012803 | EMEM + 2mM Glutamine + 1% Non-essential Aminoacids + 10 FCS | 5000 |
| MDA-MB-231 | ECACC - 92020424 | DEMEM High Glucose (4500 mg/l) + 10 FCS | 10000 |
| MDA-MB-435 | ATCC - 129 | DEMEM High Glucose (4500 mg/l) + 10 FCS | 5000 |
| MDA-MB-468 | ATCC - HTB-132 | DEMEM High Glucose (4500 mg/l) + 10 FCS | 5000 |
| MIA PaCa-2 | ECACC - 85062806 | DEMEM High Glucose (4500 mg/l) + 10 FCS | 10000 |
| NCI-H1650 | ATCC - CRL-5883 | RPMI + 2mM Glutamine + 10% FCS | 5000 |
| NCI-H1975 | ATCC - CRL-5908 | RPMI + 2mM Glutamine + 10% FCS | 5000 |
| NCI-H358 | ATCC - CRL-5807 | RPMI + 2mM Glutamine + 10% FCS | 10000 |
| OVCAR-3 | ATCC - HTB-161 | DEMEM High Glucose (4500 mg/l) + 10 FCS | 10000 |
| PANC-1 | ECACC - 87092802 | DEMEM High Glucose (4500 mg/l) + 10 FCS | 5000 |
| PC3 | ECACC - 90112714 | DEMEM High Glucose (4500 mg/l) + 10 FCS | 10000 |
| RAMOS | ECACC - 85030802 | RPMI + 2mM Glutamine + 10% FCS | 10000 |
| RCC 786-O | ATCC - CRL-1932 | DEMEM High Glucose (4500 mg/l) + 10 FCS | 10000 |
| RPMI-8226 | ECACC - 87012702 | RPMI + 2mM Glutamine + 10% FCS | 20000 |
| RWP-1 | ECACC - 87012702 | DEMEM High Glucose (4500 mg/l) + 10 FCS | 10000 |
| SK-BR-3 | ATCC - HTB-30 | DEMEM High Glucose (4500 mg/l) + 10 FCS | 10000 |
| SK-Hep-1 | ECACC - 91091986 | EMEM + 2mM Glutamine + 1% Non-essential Aminoacids + 10 FCS | 5000 |

(continued)

| Cell line | Source | Culture Medium | Cells/well |
|---|---|---|---|
| SK-Mel-28 | ATCC - HTB-72 | DEMEM High Glucose (4500 mg/l) + 10 FCS | 5000 |
| SK-OV-3 | ECACC - 91091004 | McCoy + 2mM Glutamine + 15% FCS | 5000 |
| SW480 | ECACC - 87092801 | DEMEM High Glucose (4500 mg/l) + 10 FCS | 5000 |
| T47D | ECACC - 85102201 | DEMEM High Glucose (4500 mg/l) + 10 FCS | 10000 |
| U-373 MG | ATCC - HTB17-1055 | DEMEM High Glucose (4500 mg/l) + 10 FCS | 5000 |
| U-87 MG | ECACC - 89081402 | DEMEM High Glucose (4500 mg/l) + 10 FCS | 10000 |
| Stock solutions of compounds of the invention were prepared in dimethyl sulphoxide (DMSO). | | | |

In each cell line, 72 h treatment $EC_{50}$ values were determined for each compound using AlamarBlue® and/or Hexosaminidase methods. All treatments were performed in triplicate. Experiments were carried out in 96-well cell culture plates (Cultek) and the cell lines seeded at the density mentioned in the table above and cultured at 37°C with 5% $CO_2$. After 24h, media were supplemented by 100 μl/well of diluted test compound at variable final concentrations and incubated for 72 hours. Then, AlamarBlue® Reagent (Biosource DAL1100) was added to each well and incubated at 37°C for 4 hours. Relative fluorescent intensity that correlates with the number of living cells was read in a Cytofluor plate reader (Millipore) at 535/590 nm (Excitation/emission). After reading the AlamarBlue® signal, the hexosaminidase activitity was measured according to the following protocol: medium was removed and cells were washed once with PBS. 60 μl of substrate solution (p-nitrophenol-N-acetyl-beta-D-glucosaminide 7.5 mM [Sigma N-9376], sodium citrate 0.1 M, pH 5.0, 0.25% Triton X-1 00) was added to each well and incubated at 37°C for 1-2 hours; after this incubation time, a bright yellow colour appears; then, plates could be developed by adding 90 μl of developer solution (Glycine 50 mM, pH 10.4; EDTA 5 mM), and absorbance was recorded at 405 nM.

For $EC_{50}$ determination, data were displayed graphically using GraphPad Prism 5.0, and dose-responsive curves were fitted using nonlinear regression model with a sigmoidal dose response (variable slope) equation.

Note: In all cases assays were performed in Alamar Blue with the exception of those indicated (* = Hexosaminidase assay)

| | Cell lines | $EC_{50}$ | | | | | | |
|---|---|---|---|---|---|---|---|---|
| | | Example 1 | Example 4 | Example 6 | Example 10 | Example 11 | Example 12 | MG-319 |
| Breast cancer | MDA-MB-231 | 5.1 | 2.1 | 11 | 2.8 | 1.1 | 3.2 | 18 |
| | MDA-MB-468 | 4.1 | 0.79 | 2.2 | 0.32 | 0.12 | 1.0 | 18 |
| | MCF-7 | 0.7 | 23 | 1.9 | 0.49 | 0.28 | 1.3 | 30 |
| | SK-BR-3 | 1.2 | 0.76 | 6.0* | 0.44 | 0.11 | 0.57 | 6.2 |
| | T47D | 10 | 2.1 | 21* | 0.57 | 1.3 | 7.1* | 25 |
| Colon cancer | HCT-116 | 1.9 | 1.3 | 2.2 | 0.64 | 0.28 | 0.44 | 1.8 |
| | HT-29 | 3.5 | 1.5 | 27 | 3.5 | 2.5 | 0.85 | 12.3 |
| | COLO 205 | 11 | 3.8 | 2.4 | 0.31 | 0.202 | 2.4 | 1.9* |
| | LoVo | 6.7 | 3.09 | 15 | 2.5 | 0.66 | 1.7 | 15 |
| | SW480 | 1.1 | 0.61 | 13* | 2.3* | 1.1* | 0.56 | 8.8 |
| | LS174T | 0.72 | 0.55 | 0.6 | 0.14 | 0.02 | 0.26 | 5.9 |
| Lung cancer | A549 | 7.0 | 4.0 | 12* | 2.6* | 1.3* | 5.8 | N.D. |
| | NCI-H1975 | 25 | 41 | 7.3* | 1.1* | 0.84* | 0.78 | 47 |
| | NCI-H1650 | 3.3 | 0.79 | 12* | 1.8* | 1.2* | 0.37 | 14 |
| | NCI-H358 | 0.79 | 2.4 | 3.3 | 0.7 | 0.28 | N.D. | 24 |
| Pancreatic cancer | MIA PaCa-2 | 5.7 | 3.0 | 83 | 2.4 | 0.85* | 1.9 | 22 |
| | BxPC-3 | 4.6 | 2.9 | 5.8* | 1.5* | 0.7* | 0.97 | 33 |
| | RWP-1 | 24* | 3.6 | 4.4 | 1.1 | 0.42 | N.D. | N.D. |
| | PANC-1 | N.D. | N.D. | 14 | 3.0 | 0.58 | N.D. | N.D. |
| Ovarian cancer | OVCAR-3 | 0.74 | 0.89 | 2.2* | 0.86* | 0.28* | 0.33 | 6.9 |
| | SK-OV-3 | 4.1 | 2.2 | 13 | 1.3 | 0.33 | 1.4 | 16 |
| Prostate cancer | DU-145 | 1.9 | 1.2 | 3.5 | 0.91 | 0.57 | 0.35 | 58 |
| | PC-3 | 1.5 | 2.3 | 7.1 | 1.2 | 0.68 | 0.3 | 26 |
| | LNCaP | 0.39 | 0.11 | 1.8 | 0.38 | 0.15 | 0.12 | 4.2 |
| Renal | RCC 786-O | 4.7 | 1.7 | 12 | 0.43 | 1.0 | 2.4 | 19 |

(continued)

| | Cell lines | $EC_{50}$ | | | | | | |
|---|---|---|---|---|---|---|---|---|
| | | **Example 1** | **Example 4** | **Example 6** | **Example 10** | **Example 11** | **Example 12** | **MG-319** |
| Liver cancer | HepG2 | 1.4 | 0.38 | 3.1 | 0.56 | 0.22 | 0.5 | 8.1 |
| | SK-HEP-1 | 0.49 | 0.29 | 1.9 | 0.49 | 0.007 | 0.16 | 4.2 |
| Melanoma | MDA-MB-435 | 1.0 | 0.37 | 1.1 | 0.31 | 0.17 | 0.21 | 5.3 |
| | SK-Mel-28 | 1.3 | 0.78 | 13 | 1.6 | 0.8 | 0.62 | 10 |
| Hematologic | K562 | 6.3 | 2.0 | 15 | 1.2 | 1.0 | 2.7 | 19 |
| | CCRF-CEM | 0.67 | 0.33 | 0.63 | 0.17 | 0.052 | 0.14 | 4.5 |
| | Jurkat | 2.5 | 1.7 | 2 | 0.08 | 0.04 | 0.8 | 4.7 |
| | RAMOS | 2.4 | 1.0 | 2.4 | 0.53 | 0.16 | 0.5 | 8.7 |
| Multiple mieloma | RPMI-8226 | 0.15 | 0.27 | 1.3 | 0.06 | 0.06 | 0.31 | 4.8 |
| Brain | Daoy | 1.3 | 1.3 | 3.7 | 0.78 | 0.29 | 0.57 | 41 |
| | U-373 MG | 44.0 | 7.3 | 17* | 2.8* | 1.1* | 11.0 | 92 |
| | U-87 MG | 12* | 4.2* | 12* | 0.50* | 0.89* | 2.0* | 25* |
| Epidermoid | A431 | 0.97 | 0.97 | 0.1 | 0.03* | 0.02 | 0.41 | 10 |
| Fibrosarcoma | HT-1080 | 1.7 | 1.7 | 16* | 1.4 | 0.89 | 0.46 | 8.9 |
| Cervical | HeLa | 28 | 8.3 | 54 | 13 | 3.1 | 11 | 52 |
| Osteosarcoma | 143b | 5.0 | 2.0 | 1.9 | 0.29 | 0.10 | 0.37 | 0.98 |

## Claims

1. A compound of general formula (I),

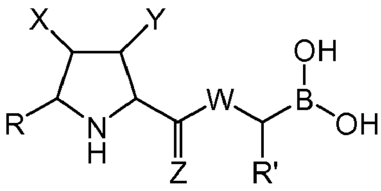

(I)

wherein:

X is an electron withdrawing group selected from $-NO_2$, $-C(O)R^1$, $-C(O)OR^1$ and $-C(O)NR^2R^3$ or forms together with Y a cycloalkyl or heterocycloalkyl group; wherein $R^1$ is an alkyl group, and $R^2$ and $R^3$ are independently selected from hydrogen and alkyl;
Y is selected from aryl, heteroaryl having at least one heteroatom, and-$C(O)OR^4$, or forms together with X a cycloalkyl or heterocycloalkyl group; wherein $R^4$ is an alkyl group;
R is selected from mono- or polycyclic aryl and heteroaryl, wherein the heteroaryl has at least one heteroatom;
Z is O or S;
W is selected from -NH-, -O- and $-CH_2-$;
R' is selected from a linear or branched alkyl and cycloalkyl,
or a stereoisomer, a solvate, a salt or prodrug thereof.

2. The compound according to claim 1, which is selected from the compounds of formula (L-Ia) and formula (D-Ia):

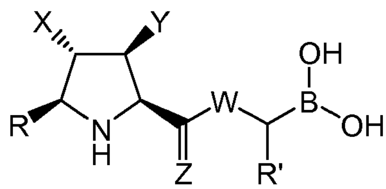

(L-Ia)

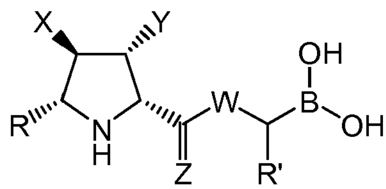

(D-Ia)

wherein:

R, R', Z and W are as defined in claim 1;
X is an electron withdrawing group selected from $-NO_2$, $C(O)R^1$, $C(O)OR^1$ and-$C(O)NR^2R^3$, wherein $R^1$, $R^2$ and $R^3$ are as defined above;
Y is selected from aryl, heteroaryl having at least one heteroatom, and-$C(O)OR^4$, wherein $R^4$ is as defined above.

3. The compound according to claim 1, which is selected from the compounds of formula (L-Ib) and formula (D-Ib):

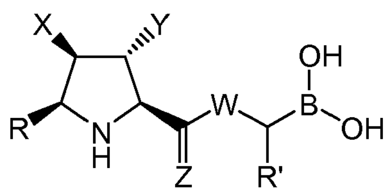

(L-Ib)

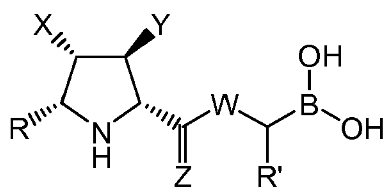

(D-Ib)

wherein:

R, R', Z and W are as defined above;
X is an electron withdrawing group selected from $-NO_2$, $C(O)R^1$, $C(O)OR^1$ and-$C(O)NR^2R^3$, wherein $R^1$, $R^2$ and $R^3$ are as defined in claim 1;
Y is selected from aryl, heteroaryl having at least one heteroatom, and-$C(O)OR^4$, wherein $R^4$ is as defined above.

**4.** The compound according to claim 1, which is selected from the compounds of formula (L-Ic) and formula (D-Ic):

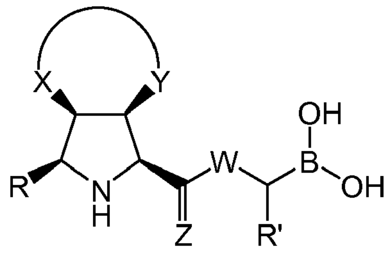

(L-Ic)

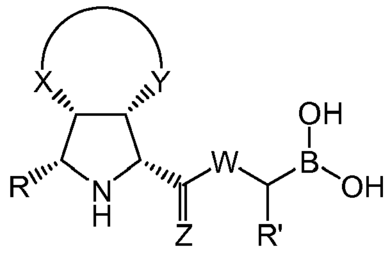

(D-Ic)

wherein

R, R', Z and W are as defined in claim 1; and
X and Y are linked to each other thus forming a cylcoalkyl or heterocycloalkyl group.

**5.** The compound according to anyone of claims 1 to 4, wherein the group W is -NH-and Z is O.

**6.** The compound according to any of claims 1 to 3 and 5, wherein X is selected from $-NO_2$, and $-C(O)OR^1$, wherein $R^1$ is an alkyl group

**7.** The compound according to any of claims 1 and 4 to 5, wherein X and Y are linked forming a heterocycloalkyl group.

**8.** The compound according to any of claims 1 to 7, wherein R' is a linear or branched alkyl group.

**9.** The compound according to claim 1 which is selected from:

- 3-Methyl-1-[4-nitro-3,5-diphenylpyrrolidine-2-carboxamido]butyl boronic acid;
- 3-Methyl-1-[4-nitro-5-phenyl-3-p-tolylpyrrolidine-2-carboxamido] butylboronic acid;
- 1-[3-(4-Fluorophenyl)-4-nitro-5-phenylpyrrolidine-2-carboxamido] -3-methylbutyl boronic acid;
- 1-[3-(4-Methoxyphenyl)-4-nitro-5-phenylpyrrolidine-2-carboxamido]-3-methylbutyl boronic acid;
- 3-Methyl-1-[4-nitro-3-(4-nitrophenyl)-5-phenylpyrrolidine-2-carboxamido]butyl boronic acid;
- 3-Methyl-1-[3-(naphthalen-2-yl)-4-nitro-5-phenylpyrrolidine-2-carboxamido]butyl boronic acid;
- 3-Methyl-1-[4-nitro-5-phenyl-3-(thiophen-2-yl)pyrrolidine-2-carboxamido]butyl boronic acid
- 1-[3-(Furan-2-yl)-4-nitro-5-phenylpyrrolidine-2-carboxamido]-3-methylbutylboronic acid;
- 3-Methyl-1-[5-(naphthalen-2-yl)-4-nitro-3-phenylpyrrolidine-2-carboxamido]butyl boronic acid;
- 3-Methyl-1-[5-(naphthalen-1-yl)-4-nitro-3-phenylpyrrolidine-2-carboxamido]butyl boronic acid;
- 3-Methyl-1-[5-(naphthalen-1-yl)-4-nitro-3-phenylpyrrolidine-2-carboxamido]butyl boronic acid;
- 1-[5-(Furan-2-yl)-4-nitro-3-phenylpyrrolidine-2-carboxamido]-3-methylbutylboronic acid;
- 3-Methyl-1-[4-nitro-3-phenyl-5-(pyridin-3-yl)pyrrolidine-2-carboxamido]butyl boronic acid;
- 1-[3,4-Bis(methoxycarbonyl)-5-phenylpyrrolidine-2-carboxamido]-3-methylbutyl boronic acid;
- 3-Methyl-1-{5-methyl-4,6-dioxo-3-phenyloctahydropyrrolo [3,4-c]pyrrole-1-carboxamido} butyl boronic acid
- 3-Methyl-1-{5-methyl-4,6-dioxo-3-phenyloctahydropyrrolo [3,4-c]pyrrole-1-carboxamido} butyl boronic acid;

or a stereoisomer, a solvate, a salt or prodrug thereof.

**10.** A compound of general formula (I) according to claim 9, selected from the group consisting of:

[1] (*R*)-3-Methyl-1-[(2*R*,3*R*,4*S*,5*R*)-4-nitro-3,5-diphenylpyrrolidine-2-carboxamido]butyl boronic acid, with the following structural formula:

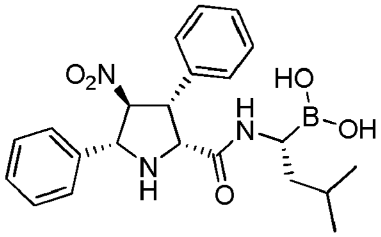

[2] (*R*)-3-Methyl-1-[(2*S*,3*S*,4*R*,5*S*)-4-nitro-3,5-diphenylpyrrolidine-2-carboxamido]butyl boronic acid, with the following structural formula:

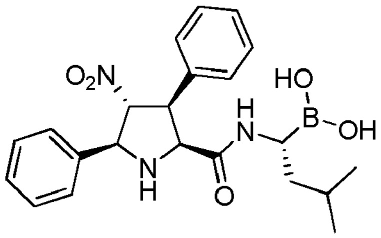

[3] (*R*)-3-Methyl-1-[(2*R*,3*S*,4*R*,5*R*)-4-nitro-3,5-diphenylpyrrolidine-2-carboxamido)butyl boronic acid, with the following structural formula:

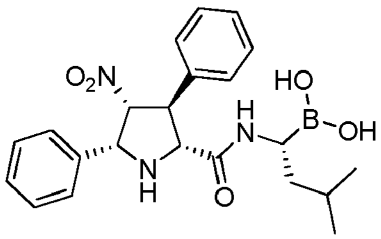

[4] (*R*)-3-Methyl-1-[(2*S*,3*R*,4*S*,5*S*)-4-nitro-3,5-diphenylpyrrolidine-2-carboxamido]butyl boronic acid, with the following structural formula:

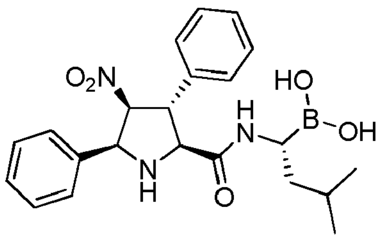

[5] (R)-3-Methyl-1-[(2R,3R,4S,5R)-4-nitro-5-phenyl-3-p-tolylpyrrolidine-2-carboxamido] butylboronic acid, with the following structural formula:

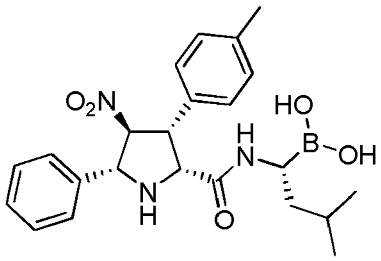

[6] (*R*)-1-[(2*R*,3*R*,4*S*,5*R*)-3-(4-Fluorophenyl)-4-nitro-5-phenylpyrrolidine-2-carboxamido] -3-methylbutylboronic acid, with the following structural formula:

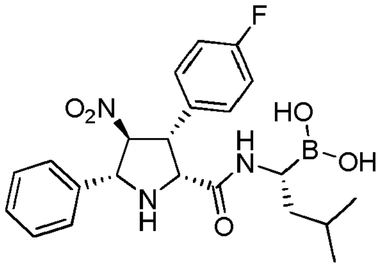

[7] (*R*)-1-[(2*R*,3*R*,4*S*,5*R*)-3-(4-Methoxyphenyl)-4-nitro-5-phenylpyrrolidine-2-carboxamido]-3-methylbutylboronic acid, with the following structural formula:

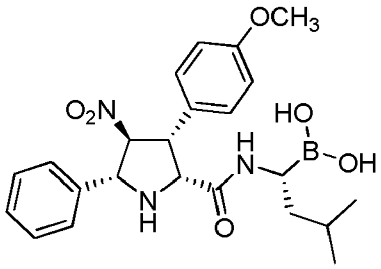

[8] (*R*)-3-Methyl-1-[(2*R*,3*R*,4*S*,5*R*)-4-nitro-3-(4-nitrophenyl)-5-phenylpyrrolidine-2-carboxamido]butylboronic acid, with the following structural formula:

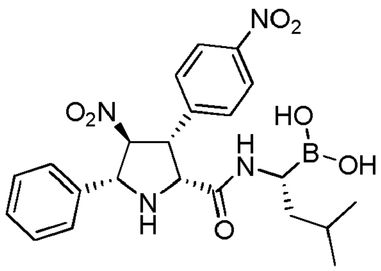

[9] (*R*)-3-Methyl-1-[(2*R*,3*R*,4*S*,5*R*)-3-(naphthalen-2-yl)-4-nitro-5-phenylpyrrolidine-2-carboxamido]butylboronic acid, with the following structural formula:

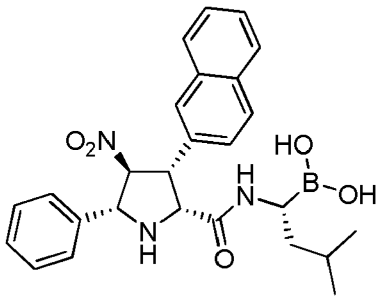

[10] (*R*)-3-Methyl-1-[(2*R*,3*R*,4*S*,5*R*)-4-nitro-5-phenyl-3-(thiophen-2-yl)pyrrolidine-2-carboxamido]butylboronic acid, with the following structural formula:

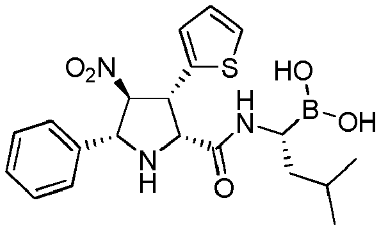

[11] (*R*)-1-[(2*R*,3*R*,4*S*,5*R*)-3-(Furan-2-yl)-4-nitro-5-phenylpyrrolidine-2-carboxamido]-3-methylbutylboronic acid, with the following structural formula:

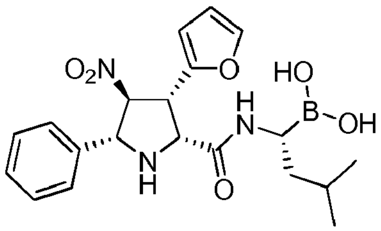

[12] (*R*)-3-Methyl-1-[(2*R*,3*R*,4*S*,5*R*)-5-(naphthalen-2-yl)-4-nitro-3-phenylpyrrolidine-2-carboxamido]butylboronic acid, with the following structural formula:

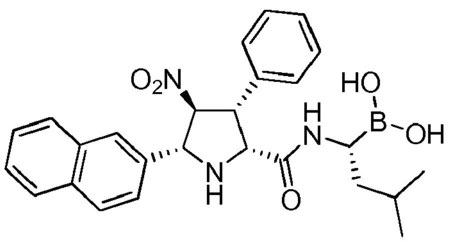

[13] (*R*)-3-Methyl-1-[(2*R*,3*R*,4*S*,5*R*)-5-(naphthalen-1-yl)-4-nitro-3-phenylpyrrolidine-2-carboxamido]butylboronic acid, with the following structural formula:

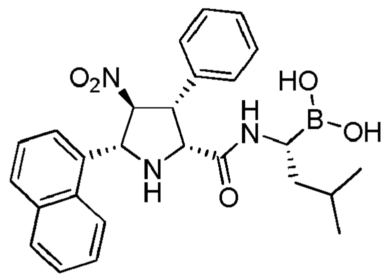

[14] (*R*)-3-Methyl-1-[(2*R*,3*S*,4*R*,5*R*)-5-(naphthalen-1-yl)-4-nitro-3-phenylpyrrolidine-2-carboxamido]butylboronic acid, with the following structural formula:

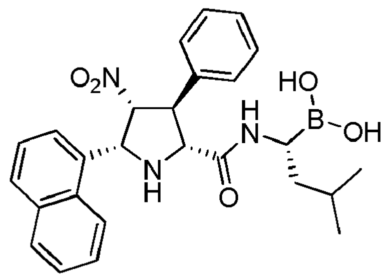

[15] (*R*)-1-[(2*R*,3*R*,4*S*,5*S*)-5-(Furan-2-yl)-4-nitro-3-phenylpyrrolidine-2-carboxamido]-3-methylbutylboronic acid, with the following structural formula:

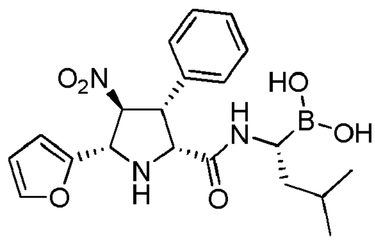

[16] (*R*)-3-Methyl-1-[(2*R*,3*R*,4*S*,5*R*)-4-nitro-3-phenyl-5-(pyridin-3-yl)pyrrolidine-2-carboxamido]butylboronic acid, with the following structural formula:

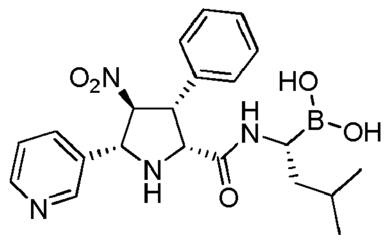

[17] (*R*)-1-[(2*S*,3*S*,4*S*,5*R*)-3,4-Bis(methoxycarbonyl)-5-phenylpyrrolidine-2-carboxamido]-3-methylbutylboronic acid, with the following structural formula:

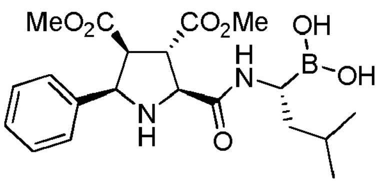

[18] (*R*)-3-Methyl-1-{(1*S*,3*R*,3a*S*,6a*R*)-5-methyl-4,6-dioxo-3-phenyloctahydropyrrolo [3,4-c]pyrrole-1-carboxamido}butylboronic acid, with the following structural formula:

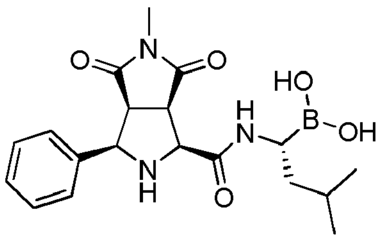

[19] (*R*)-3-Methyl-1-{(1*R*,3*S*,3a*R*,6a*S*)-5-methyl-4,6-dioxo-3-phenyloctahydropyrrolo [3,4-c]pyrrole-1-carboxamido}butylboronic acid, with the following structural formula:

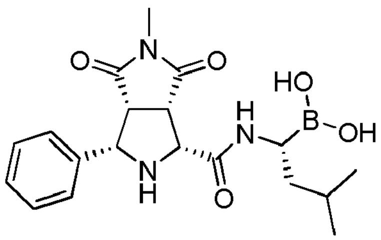

or a stereoisomer, a solvate, a salt or prodrug thereof.

**11.** A process for the preparation of a compound of general formula (I) as defined in any of claims 1 to 10, wherein X, Y, R, R', Z and W have the meaning given in claim 1 which comprises:

a) reacting an alkene of general formula (IIa) or (IIb),

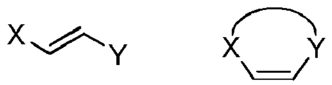

(IIa) (IIb)

wherein X and Y have the meaning given above;
with an imine of general formula (III),

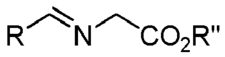

(III)

wherein R has the meaning given above and R" is a linear or branched alkyl group selected from methyl, ethyl and tert-butyl;

in the presence of an organic or inorganic base, an aprotic solvent and a metallic salt to afford a compound of formula (IV):

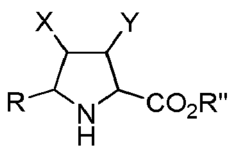

(IV)

b) reacting a mixture of compound of formula (IV) with a mixture comprising an organic solvent and:

i) an aqueous solution of an alkali or alkaline earth hydroxide; or
ii) a trialkyltin hydroxide; or
iii) an organic acid,
to afford a compound of formula (VI)

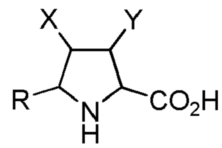

(VI)

c) reacting the compound of formula (VI) with aboronic ester of formula (VII):

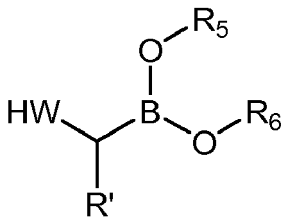

(VII)

(VII)

wherein

R' and W have the meaning given above,
$R^5$ and $R^6$ are independently an alkyl group or together form an alkylene group, a cycloalkylene group or an arylene group;
the group WH can be neutral or forming a salt,
in an organic solvent in the presence of an organic base and a coupling agent,
to afford an intermediate boronic ester;

d) deprotecting the intermediate boronic ester by treatment with isobutylboronic acid and an aqueous solution of hydrochloric acid, to afford the compound of formula (I) wherein Z is O; and
e) reacting the compound of formula (I) obtained in step d) with a Lawesson reactive or a derivative thereof to afford the compound of formula (I) wherein Z is S.

**12.** The process according to claim 11, wherein step a) is carried out in the presence of a chiral catalyst.

**13.** A compound of formula (I) as defined in any of claims 1 to 10, or a stereoisomer or a pharmaceutically acceptable solvate or a salt or prodrug thereof, for its use as a medicament.

**14.** A compound of formula (I) as defined in any of claims 1 to 10, or a stereisomer, or a pharmaceutically acceptable solvate or a salt or prodrug thereof, for its use in the treatment of a disease or condition selected from the group consisting of cancer, hematological malignancy, proliferative diseases, autoimmune diseases, neurodegenerative diseases and viral infections.

**15.** A pharmaceutical composition that comprises at least a compound of formula (I) as defined in any of claims 1 to 10, or a stereisomer, or a pharmaceutically acceptable solvate or a salt or prodrug thereof, and at least a pharmaceutically acceptable excipient.

Europäisches Patentamt
European Patent Office
Office européen des brevets

# EUROPEAN SEARCH REPORT

Application Number
EP 14 38 2058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 96/13266 A1 (PROSCRIPT INC [US]) 9 May 1996 (1996-05-09) * pages 88-89; examples MG-359, MG-363 * | 1-15 | INV. C07F5/02 A61P35/00 A61K31/69 |
| A | MENG LEI ET AL: "Pharmacophore Modeling, Docking Studies, and Synthesis of Novel Dipeptide Proteasome Inhibitors Containing Boron Atoms", JOURNAL OF CHEMICAL INFORMATION AND MODELING, vol. 49, no. 9, 28 September 2009 (2009-09-28), pages 2092-2100, XP055111730, ISSN: 1549-9596, DOI: 10.1021/ci900225s * page 2096 * | 1-15 | |
| A | US 7 446 125 B2 (COSSIO FERNANDO P [ES] ET AL PEDRO COSSIO FERNANDO [ES] ET AL) 4 November 2008 (2008-11-04) * claim 1 * | 1-15 | |
| | | | TECHNICAL FIELDS SEARCHED (IPC) |
| | | | C07F A61P A61K |

The present search report has been drawn up for all claims

2

| Place of search | Date of completion of the search | Examiner |
|---|---|---|
| The Hague | 3 April 2014 | Duval, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 38 2058

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2014

| Patent document cited in search report | Publication date | Patent family member(s) | Publication date |
|---|---|---|---|
| WO 9613266 A1 | 09-05-1996 | AT 241631 T | 15-06-2003 |
| | | AT 314378 T | 15-01-2006 |
| | | AT 411324 T | 15-10-2008 |
| | | AU 710564 B2 | 23-09-1999 |
| | | CA 2203936 A1 | 09-05-1996 |
| | | CA 2496538 A1 | 09-05-1996 |
| | | CH 0788360 H1 | 15-03-2006 |
| | | CN 1168633 A | 24-12-1997 |
| | | CN 101077875 A | 28-11-2007 |
| | | CY 2484 B1 | 03-06-2005 |
| | | DE 69530936 D1 | 03-07-2003 |
| | | DE 69530936 T2 | 26-02-2004 |
| | | DE 69534727 T2 | 14-09-2006 |
| | | DE 122004000025 I1 | 30-09-2004 |
| | | DE 122004000025 I2 | 07-09-2006 |
| | | DK 0788360 T3 | 22-09-2003 |
| | | DK 1312609 T3 | 15-05-2006 |
| | | DK 1627880 T3 | 08-12-2008 |
| | | EP 0788360 A1 | 13-08-1997 |
| | | EP 1312609 A1 | 21-05-2003 |
| | | EP 1627880 A1 | 22-02-2006 |
| | | EP 1997823 A1 | 03-12-2008 |
| | | ES 2199257 T3 | 16-02-2004 |
| | | ES 2254803 T3 | 16-06-2006 |
| | | ES 2314540 T3 | 16-03-2009 |
| | | FI 971746 A | 06-06-1997 |
| | | FI 20041415 A | 03-11-2004 |
| | | HK 1002059 A1 | 05-09-2003 |
| | | HK 1087714 A1 | 22-05-2009 |
| | | IL 115790 A | 01-12-2002 |
| | | IL 133831 A | 28-03-2004 |
| | | IL 137726 A | 31-08-2004 |
| | | JP 3717934 B2 | 16-11-2005 |
| | | JP H10510245 A | 06-10-1998 |
| | | LU 91083 I2 | 09-08-2004 |
| | | NL 300151 I1 | 02-08-2004 |
| | | NO 971929 A | 12-06-1997 |
| | | NZ 296717 A | 29-11-1999 |
| | | NZ 337211 A | 22-12-2000 |
| | | PT 788360 E | 31-10-2003 |
| | | PT 1312609 E | 31-05-2006 |
| | | PT 1627880 E | 23-01-2009 |
| | | US 5780454 A | 14-07-1998 |
| | | US 6066730 A | 23-05-2000 |
| | | US 6083903 A | 04-07-2000 |
| | | US 6297217 B1 | 02-10-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 38 2058

This annex lists the patent family members relating to the patent documents cited in the above-mentioned European search report. The members are as contained in the European Patent Office EDP file on
The European Patent Office is in no way liable for these particulars which are merely given for the purpose of information.

03-04-2014

| Patent document cited in search report | Publication date | Patent family member(s) | Publication date |
|---|---|---|---|
| | | US 6465433 B1 | 15-10-2002 |
| | | US 6617317 B1 | 09-09-2003 |
| | | US 2002173488 A1 | 21-11-2002 |
| | | US 2003199561 A1 | 23-10-2003 |
| | | US 2004167332 A1 | 26-08-2004 |
| | | US 2006122390 A1 | 08-06-2006 |
| | | US 2007282100 A1 | 06-12-2007 |
| | | US 2008132678 A1 | 05-06-2008 |
| | | US 2009247731 A1 | 01-10-2009 |
| | | US 2011306560 A1 | 15-12-2011 |
| | | US 2013310320 A1 | 21-11-2013 |
| | | WO 9613266 A1 | 09-05-1996 |
| US 7446125 B2 | 04-11-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9613266 A, Adams


### Non-patent literature cited in the description


- **GROLL et al.** *ChemBioChem,* 2005, vol. 6, 222-256
- **CIECHANOVER.** *Angew. Chem. Int. Ed.,* 2005, vol. 44, 5944-5967
- **BIEL et al.** *Angew. Chem. Int. Ed.,* 2004, vol. 43, 6414-6416
- **MARQUES et al.** *Chem. Rev.,* 2009, vol. 109, 1509-1536
- **BORISSENKO ; GROLL.** *Chem. Rev.,* 2007, vol. 107, 687-717
- **GOLDBERG.** *J. Cell Biol.,* 2012, vol. 199, 583-588
- **MOORE et al.** *Curr. Opin. Chem. Biol.,* 2008, vol. 12, 434-440
- **CVEK ; DVORAK.** *Drug Discov. Today,* 2008, vol. 13, 716-722
- **BETTIGNIES ; COUX.** *Biochimie,* 2010, vol. 92, 1530-1545
- **GUÉDAT ; COLLAND.** *BMC Biochem.,* 2007, vol. 8 (1), 14
- **D'ALESSANDRO et al.** *Recent Pat. Anti-Cancer Drug Discov.,* 2009, vol. 4, 73-82
- **DICK ; FLEMING.** *Drug Discov. Today,* 2010, vol. 15, 243-249
- **RENTSCH et al.** *Angew. Chem. Int. Ed.,* 2013, vol. 52, 5450-5488
- **KISSELEV et al.** *Chem. Biol.,* 2012, vol. 19, 99-115
- **MOLINEAUX.** *Clin. Cancer Res.,* 2012, vol. 18, 15-20
- **KALE ; MOORE.** *J. Med. Chem.,* 2012, vol. 55, 10317-10327
- **GROLL et al.** *J. Pept. Sci.,* 2009, vol. 15, 58-66
- **ARASTU-KAPUR et al.** *Clin. Cancer Res.,* 2011, vol. 17, 2734-2743
- **GROLL et al.** *Structure,* 2006, vol. 14, 451-456
- **CONDE et al.** *Chem. Sci.,* 2012, vol. 3, 1486-1491
- **CASTELLÓ et al.** *Org. Lett.,* 2013, vol. 15, 2902-2905
- **KROGSGAARD-LARSEN et al.** Textbook of Drug design and Discovery. Taylor & Francis, April 2002
- **KALETA, Z et al.** *Org. Lett.,* 2006, vol. 8, 1625-1628
- **JONES, B. A. et al.** *Chem. Rev. (Review,* vol. 84 (84), 17
- **SHABANA, R. et al.** *Nouveau Journal de Chimie,* 1980, 47
- **E.W. MARTIN.** Remington's Pharmaceutical Sciences. 2005
- **ROWE C. R. ; PAUL J. S. ; MARIAN E. Q.** Handbook of Pharmaceutical Excipients